(19) Europäisches Patentamt / European Patent Office / Office européen des brevets

(11) **EP 3 909 593 A1**

(12) **EUROPEAN PATENT APPLICATION**

(43) Date of publication:
**17.11.2021 Bulletin 2021/46**

(51) Int Cl.:
**A61K 35/28** (2015.01)   **A61K 9/00** (2006.01)
**A61P 17/02** (2006.01)

(21) Application number: **20175024.7**

(22) Date of filing: **15.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Rigshospitalet**
  **2100 Copenhagen Ø (DK)**
• **Københavns Universitet**
  **1165 Copenhagen K (DK)**

(72) Inventors:
• **Rangatchew, Filip Kiril**
  **2450 Copenhagen SV (DK)**
• **Holmgaard, Rikke**
  **2200 Copenhagen N (DK)**
• **Munthe-Fog, Lea**
  **2100 Copenhagen Ø (DK)**
• **Drzewiecki, Krzysztof T.**
  **2920 Charlottenlund (DK)**

(74) Representative: **Inspicos P/S**
  **Kogle Allé 2**
  **2970 Hørsholm (DK)**

(54)   **STEM CELLS FOR TREATING SKIN LESIONS**

(57)   Methods of treating or preventing burn wounds and other skin lesions, disorders or diseases using stem cells, as well as methods of administering stem cells to the skin by intradermal or transdermal injection.

FIG. 1

**Description**

FIELD OF THE INVENTION

The present invention relates to compositions comprising stem cells, preferably mesenchymal stem cells (MSCs), for use in methods of treating burn wounds as well as in the treatment and prevention of other skin lesions, disorders or diseases.

BACKGROUND OF THE INVENTION

Globally, the incidence of burns requiring medical attention is nearly 11 million people, making it the fourth most common injury (Peck, Burns 2011;37:1087-100). Patients suffering from devastating consequences of severe burns with functional, aesthetic and psychosocial sequelae are a testament to a suboptimal conventional therapy. MSCs, including adipose-derived MSCs (ASCs), have been proposed as a treatment option for several diseases and conditions (WO 2017/068140; WO 2014/203267, WO 2017/144552, and Isakson et al., 2015). These include burn wounds, although human studies have so far been limited (Francis et al., 2019; Golchin et al., 2019; Rasulov et al., 2005; Mansilla et al., 2015; Abo-Elkheir et al., 2017; WO 2006/074075; WO 2016/054592, US 2007/0274967).
The skin is, however, on most places of the human body only a few millimetres thick. This makes precise and reliable injectable drug delivery at a burn wound or other target site a time consuming and strenuous task, especially if larger skin areas need to be treated. While automated injection devices have been proposed as being suitable for cells (e.g., WO 2007/042818) and shown useful for some types of cells (Leoni et al., 2017), other studies have indicated that ejection of MSCs using an automated injectable cell delivery devices at high cell concentrations may significantly limit cell viability (Kondziolka et al., 2011), emphasizing the importance of characterizing cellular health post-injection (Amer et al., 2017). Moreover, factors such as needle clogging, delivery efficiency and reflux may affect the dosage delivered and thereby therapeutic efficacy and reproducibility of stem cell therapies of burn wounds and other skin conditions.
Despite these and other advances in the art, there is still a need for safe, reproducible and clinically effective therapies for treating burn wounds and other lesions, disorders and diseases of the skin, particularly when stem cells are used as the therapeutic agent. It is an object of embodiments of the invention to provide such therapies.

SUMMARY OF THE INVENTION

It has been found by the present inventors that suspensions of human stem cells can be used in the treatment or prevention of a skin lesion in a human subject by administering the suspensions by intradermal or transdermal injection at a higher angle to the skin surface than those normally used, typically an angle of at least about 60 degrees.
It has also been found by the present inventors that allogeneic ASCs can be used in treating a burn wound in the skin of in a human subject, typically at dosages of from about $1 \times 10^4$ to about $1 \times 10^6$ ASCs per $cm^2$ of a burn wound.
Accordingly, the following particular aspects and embodiments of the invention are provided:

1. A composition comprising a suspension of human stem cells for use in the treatment of a skin lesion in a human subject, comprising administering the composition to the skin lesion by intradermal or transdermal injection using one or more injection needles inserted into the skin at an angle of at least about 60 degrees to the skin surface.

2. A composition comprising a suspension of human stem cells for use in the prevention of a skin lesion in a human subject, comprising administering the composition to a skin area at risk for a skin lesion by intradermal or transdermal injection using one or more injection needles inserted into the skin at an angle of at least about 60 degrees to the skin surface.

3. The composition for the use according to any one of the preceding embodiments, wherein the human stem cells are mesenchymal stem cells (MSCs), optionally derived from adipose tissue, bone marrow, umbilical cord tissue and/or umbilical cord blood.

4. The composition for the use according to any one of the preceding embodiments, wherein the stem cells are adipose tissue-derived mesenchymal stem cells (ASCs).

5. The composition for the use according to any one of the preceding embodiments, wherein the skin lesion is caused by an injury, or a disease or disorder selected from an inflammatory disease or disorder, an autoimmune disease or disorder, an allergic or hypersensitivity disease or disorder, a degenerative disease or disorder, an ischemic disease or disorder, a rheumatic disease or disorder, a metabolic disease or disorder, an endocrine disease or disorder or a benign neoplastic disease or disorder.

6. The composition for the use according to embodiment 5, wherein the disease or disorder is selected from alopecia, epidermolysis bullosa, dermatitis, scleroderma, diabetes, lupus, ischemia, vasculitis, psoriasis, rosacea, atopic dermatitis, lichen sclerosus, lichen planus and lymphedema.

7. The composition for the use according to any one of the preceding embodiments, wherein the skin lesion is selected from a wound, an ulcer, a scar and a rash.

8. The composition for the use according to any one of the preceding embodiments, wherein the skin lesion is a burn wound.

9. The composition for the use according to embodiment 8, wherein the burn wound has not been subjected to prior escharectomy or necrectomy.

10. A composition comprising allogeneic ASCs for use in treating a burn wound in the skin of in a human subject, comprising administration of from about $1 \times 10^4$ to about $1 \times 10^6$ ASCs per $cm^2$ of a burn wound, without prior escharectomy or necrectomy.

11. The composition for the use according to embodiment 10, wherein the composition is administered by intradermal injection, subcutaneous injection, or topical application.

12. The composition for the use according to any one of embodiments 8 to 11, wherein the composition is administered within no more than about 36 hours from the burn injury.

13. The composition for the use according to any one of embodiments 8 to 12, wherein the burn wound is a partial-thickness burn wound, such as a deep partial-thickness burn wound at risk for progression into a full-thickness burn wound, or a superficial partial-thickness burn wound at risk for progressing into at least a deep-partial-thickness burn wound.

14. The composition for the use according to any one of embodiments 8 to 13, wherein the burn wound is a thermal burn wound.

15. The composition for the use according to any one of embodiments 8 to 14, wherein the use results in:

    (a) a reduced extent of tissue damage or necrosis of the burn wound;
    (b) a reduced extent of apoptosis in the burn wound;
    (c) a reduced extent of local inflammation;
    (d) an increased revascularisation and/or angiogenesis of the burn wound;
    (e) an increased blood perfusion of the burn wound;
    (f) an increased and/or accelerated regeneration of skin appendages and normal anatomical structures of the skin of the burn wound;
    (g) an increased degree and/or rate of healing of the burn wound;
    (h) a reduced need for later skin grafting;
    (i) a reduced extent of scarring and/or fibrosis of the burn wound;
    (j) an increased quality of the scar from the burn wound; and
    (k) a combination of any two or more of (a) to (j).

16. The composition for the use according to any one of the preceding embodiments, wherein the composition is administered to a skin area of at least 1 $cm^2$, such as at least 2 $cm^2$, such as at least 4 $cm^2$, such as at least 10 $cm^2$, such as at least 20 $cm^2$, such as at least 30 $cm^2$, such as at least 40 $cm^2$.

17. The composition for the use according to any one of the preceding embodiments, wherein the composition is administered by intradermal or subcutaneous injection using one or more injection needles.

18. The composition for the use according to embodiment 17, wherein the one or more injection needles are inserted into the skin at an angle of at least about 70 degrees to the skin surface, such as at least about 75 degrees to the skin surface, such as at least about 80 degrees to the skin surface, such as at least about 85 degrees to the skin surface, such as substantially perpendicular to the skin surface.

19. The composition for the use according to any one of embodiments 17 and 18, wherein the composition is administered at between 2 to 27 needle insertion sites per $cm^2$ of skin, such as between 3 and 18 insertion sites per $cm^2$ of skin, such as between 5 and 15 insertion sites per $cm^2$ of skin, such as between 7 and 11 insertion sites per $cm^2$ of skin.

20. The composition for the use according to any one of embodiments 17 to 19, wherein the composition is administered using a delivery device comprising plurality of substantially parallel injection needles, wherein the device is adapted to deliver a liquid formulation, optionally a viscous liquid formulation, through the plurality of substantially parallel injection needles when inserted into the skin.

21. The composition for the use of embodiment 20, wherein the delivery device is adapted to keep the skin area in and around the insertion site of each injection needle taut during at least the needle insertion and injection phases, optionally by creating a vacuum, suction or mechanical force to gently lift the skin towards a surface comprising an opening from which the injection needle emerges.

22. The composition for the use of embodiment 21, wherein the device comprises a vacuum chamber comprising at least the tip of each injection needle.

23. The composition for the use according to any one of embodiments 20 to 22, comprising administering the composition by a method comprising the steps of:

(a) applying the device to a predetermined area of the skin, wherein the device is loaded with a volume of the composition and activated to create a vacuum, suction or mechanical force to gently lift the skin towards a plurality of surfaces, each surface comprising an opening from which an injection needle can emerge;

(b) activating the device to

i. insert the tip of each injection needle into a predetermined injection needle insertion/injection depth in the skin; and

ii. inject from each injection needle a predetermined volume of the composition;

(c) optionally, repeating steps (a) and (b) at an adjacent or other predetermined area of the skin.

24. The composition for the use according to any one of embodiments 17 to 23, wherein the composition is administered at a needle insertion/injection depth of at least about 0.6 mm from the skin surface, such as at least 1 mm from the skin surface, such as at least 1.5 mm from the skin surface.

25. The composition for the use according to any one of embodiments 17 to 24, wherein the composition is administered by intradermal or subcutaneous injection at a volume per injection needle insertion site of at least about 0.5 microliter ($\mu$L), at most about 20 $\mu$L, or both.

26. The composition for the use according to any one of embodiments 17 to 25, wherein the composition is administered at a volume per injection needle insertion site of from about 1 $\mu$L to about 10 $\mu$L, such as from about 2 $\mu$L to about 5 $\mu$L.

27. The composition for the use according to any one embodiments 17 to 26, wherein the composition is administered at an injection rate per needle of no more than about 0.5 mL/min, such as no more than about 0.4 mL/min, such as no more than about 0.3 mL/min, such as no more than about 0.25 mL/min, such as no more than about 0.22 mL/min.

28. The composition for the use according to any one of embodiments 17 to 27, wherein, after the injection, each needle remains inserted into the skin for a period of at least 3 seconds, such as at least 4 seconds, such as at least 5 seconds, such as at least 6 seconds.

29. The composition for the use according to any one of embodiments 17 to 28, wherein the composition is administered at a dosage per injection needle insertion site of at least about $1 \times 10^3$ stem cells, at most about $1 \times 10^6$

stem cells or both.

30. The composition for the use according to embodiment 29, wherein the composition is administered at a dosage per injection needle insertion site of at least about $1 \times 10^3$ stem cells and at most about $2 \times 10^5$ stem cells, such as at most about $1 \times 10^5$ stem cells.

31. The composition for the use according to any one of embodiments 17 to 30, wherein the composition is administered via one or more injection needles having an inner diameter of at least about 0.1 mm, at most about 0.5 mm, or both.

32. The composition for the use according to embodiment 31, wherein the composition is administered via one or more injection needles having an inner diameter of more than about 0.1 mm, optionally having a Gauge (G) selected from 25G, 26G, 27G, 28G, 29G, 30G, 31G, 32G and 33G.

33. The composition for the use according to any one of embodiments 17 to 32, wherein the length of the injection needle is at most about 3 cm, such as a length of at most 2 cm, such as a length of at most about 1.8 cm.

34. The composition for the use according to any one of embodiments 17 to 33, wherein the composition is administered by intradermal injection.

35. The composition for the use according to any one of the preceding embodiments, wherein the concentration of the stem cells in the composition is at least $1 \times 10^6$ per mL, at most about $1 \times 10^8$ per mL or both.

36. The composition for the use according to any one of the preceding embodiments, wherein the concentration of the stem cells in the composition is from about $1 \times 10^7$ to about $5 \times 10^7$ per mL, such as from about $1 \times 10^7$ to about $4 \times 10^7$ per mL.

37. The composition for the use according to any one of the preceding embodiments, wherein the composition is prepared by suspending the stem cells in a protein-free cryoprotectant at a concentration of at least about $1.5 \times 10^7$ stem cells per mL, such as a concentration of about $2.2 \times 10^7$ stem cells per mL.

38. The composition for the use according to embodiment 36, wherein the protein-free cryoprotectant comprises dimethylsulphoxide (DMSO) at a concentration of about 5% to about 15% (v/v), such as about 5% or about 10% (v/v).

39. The composition for the use according to embodiment 37, wherein the protein-free cryoprotectant further comprises Trolox (6-hydroxy-2,5,7,8-tetramethylchroman-2-carboxylic acid), $Na^+$, $K^+$, $Ca^{2+}$, $Mg^{2+}$, $Cl^-$, $H_2PO_4^-$, HEPES, lactobionate, sucrose, mannitol, glucose, dextran-40, adenosine and glutathione.

40. The composition for the use according to any one of the preceding embodiments, wherein the composition comprises an ASC population wherein at least about 80% express CD90, CD73, CD13, CD105, CD29, CD166, CD10, CD140b, CD160, CD204, CD272, CD44, CD49a, CD54, CD9, Galectin 3, Galectin 9, HLA-G and LTβR and wherein at most about 15% of the ASC population express CD45, CD19, CD14, CD106, CD31 and CD36.

41. The composition for the use according to any one of the preceding embodiments, wherein the composition is obtained or obtainable by a process comprising the steps of

(a) adding the stromal vascular fraction (SVF) of a lipoaspirate collected from a human donor to a bioreactor wherein at least one surface is pre-treated to promote adhesion of human stem cells;
(b) in the bioreactor, cultivating adherent cells to confluence in a serum-free culture medium supplemented with human platelet lysate;
(c) detaching the adherent cells;
(d) freezing the detached cells in a cryoprotectant at a concentration of at least $1 \times 10^6$ cells/mL;
(e) thawing the frozen cells and repeating steps (ii) and (iii) at least once,
(f) freezing the detached cells at a concentration of at least $1.5 \times 10^7$ cells/mL; and
(g) thawing the frozen composition.

42. A method of treating a skin lesion in a subject in need thereof, the method comprising administering an effective amount of a composition comprising a suspension of stem cells to the skin lesion by intradermal or transdermal injection using one or more injection needles inserted into the skin at an angle of at least about 60 degrees to the skin surface.

43. A method of preventing a skin lesion in a subject in need thereof, the method comprising administering an effective amount of a composition comprising a suspension of stem cells to a skin area at risk for a skin lesion by intradermal or transdermal injection using one or more injection needles inserted into the skin at an angle of at least about 60 degrees to the skin surface.

44. A method of treating a skin disease or disorder in a subject in need thereof, the method comprising administering an effective amount of a composition comprising a suspension of stem cells to a skin lesion associated with the skin disease or disorder by intradermal or transdermal injection using one or more injection needles inserted into the skin at an angle of at least about 60 degrees to the skin surface.

45. A method of treating a skin disease or disorder in a subject in need thereof, the method comprising administering an effective amount of a composition comprising a suspension of stem cells to a skin area at risk for a skin lesion associated with the skin disease or disorder by intradermal or transdermal injection using one or more injection needles inserted into the skin at an angle of at least about 60 degrees to the skin surface.

46. A method of administering a composition comprising stem cells into the dermis or hypodermis of a subject, the method comprising administering a suspension of stem cells to a predetermined skin area by intradermal or transdermal injection using one or more injection needles inserted into the skin at an angle of at least about 60 degrees to the skin surface.

47. A method of treating a burn wound in a subject in need thereof, the method comprising administering an effective amount of a composition comprising a suspension of stem cells to a burn wound by intradermal or transdermal injection using one or more injection needles inserted into the skin at an angle of at least about 60 degrees to the skin surface.

48. A method of reducing the extent of a tissue damage or necrosis of a burn wound, reducing the extent of apoptosis in a burn wound, reducing the extent of local inflammation in a burn wound, increasing the revascularisation of a burn wound, increasing the blood perfusion of a burn wound, increasing the regeneration of skin appendages and normal anatomical structures of the skin of the burn wound, increasing the degree and/or rate of healing of a burn wound, reducing the need for later skin grafting of a burn wound, reducing the extent of scarring/fibrosis of a burn wound and/or improving the quality of the scar from a burn wound, the method comprising administering an effective amount of a composition comprising a suspension of stem cells to a burn wound by intradermal or transdermal injection using one or more injection needles inserted into the skin at an angle of at least about 60 degrees to the skin surface.

49. A method of treating a burn wound in the skin of in a human subject, the method comprising administering a composition comprising allogeneic ASCs at a dosage of from about $1 \times 10^4$ to about $1 \times 10^6$ ASCs per cm$^2$ of the burn wound, without prior escharectomy or necrectomy of the burn wound.

50. A method of reducing scar formation from a burn wound in a subject in need thereof, the method comprising administering a composition comprising allogeneic ASCs at a dosage of from about $1 \times 10^4$ to about $1 \times 10^6$ ASCs per cm$^2$ of the burn wound, without prior escharectomy or necrectomy of the burn wound.

51. A method of preventing the progression of a partial-thickness burn wound into a deep partial-thickness burn wound and/or a full-thickness burn wound, the method comprising administering a composition comprising allogeneic ASCs at a dosage of from about $1 \times 10^4$ to about $1 \times 10^6$ ASCs per cm$^2$ of the burn wound, without prior escharectomy or necrectomy of the burn wound.

52. The method of any one of embodiments 42 to 51, further comprising the features of any one or more of embodiments 1 to 41.

These and other aspects and embodiments are described in more detail below and set forth in the appended claims.

FIGURE LEGENDS

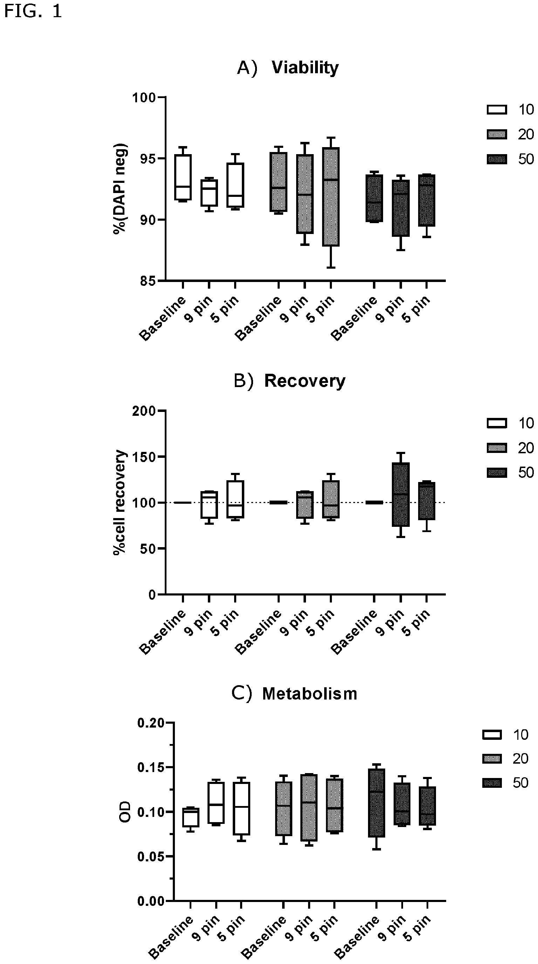

**Figure 1.** No significant difference was observed in post-ejectional perceived viability (A), recovery (B) and metabolism (C) vs baseline of each cell concentration (10, 20 and 50 x10$^6$ ASCs/ml), ejected through either a 9- or 5-pin head. For details, see Example 1.

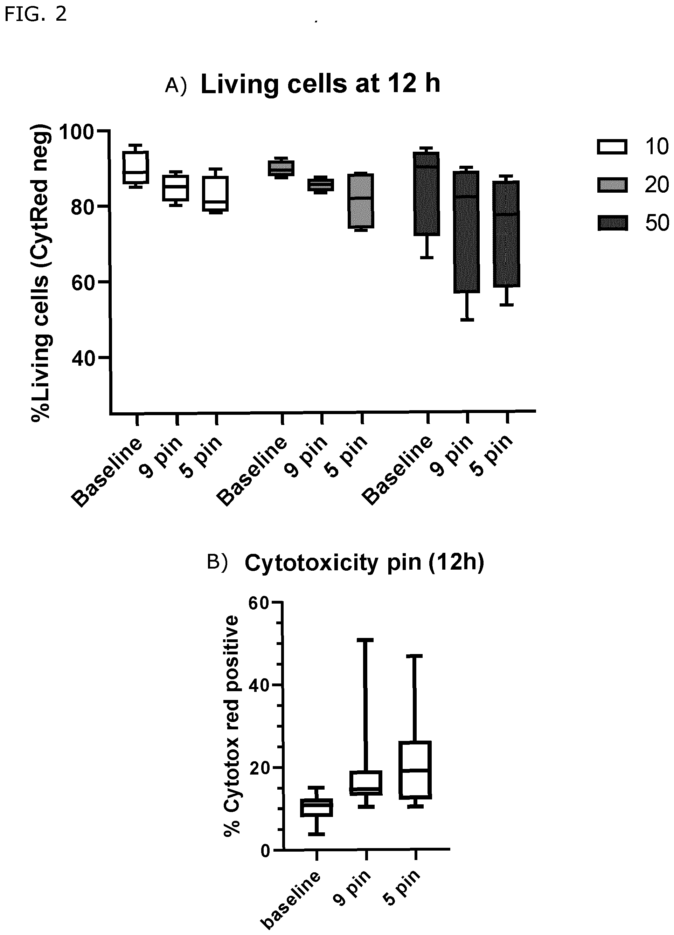

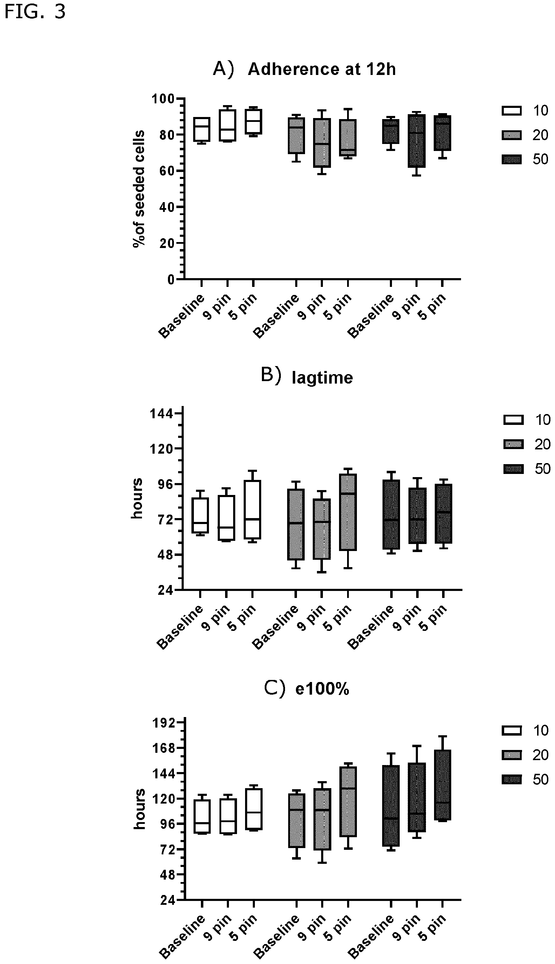

**Figure 2.** The long-time cell viability (accumulated cell death over 12 hours) declined significantly regardless of cell concentration (10, 20 or 50 x10$^6$ ASCs/ml) when ejected through either the 9-pin (p=0.0043) or 5-pin (p=0.0002) head when compared with baseline (A) (B) (see also Table 1). The higher cell concentration seemed to be less viable when ejected through cannulas. The mean long-time cell viability appeared lower for 5- vs. 9-pin, supported by longer time to reach confluency, although not significant as shown in Fig 3. However, the mean long-term viability result was within the defined range of acceptance (post-thaw viability >70%).

**Figure 3.** Although no significant difference was observed in adherence (A) and hours in lag phase (B) when culturing the dispensed cells (10, 20 and 50 x10$^6$ ASCs/ml), a tendency to prolonged time to reach confluence (e100%, estimated confluency) (C) were observed in cells ejected through the 5-pin head.

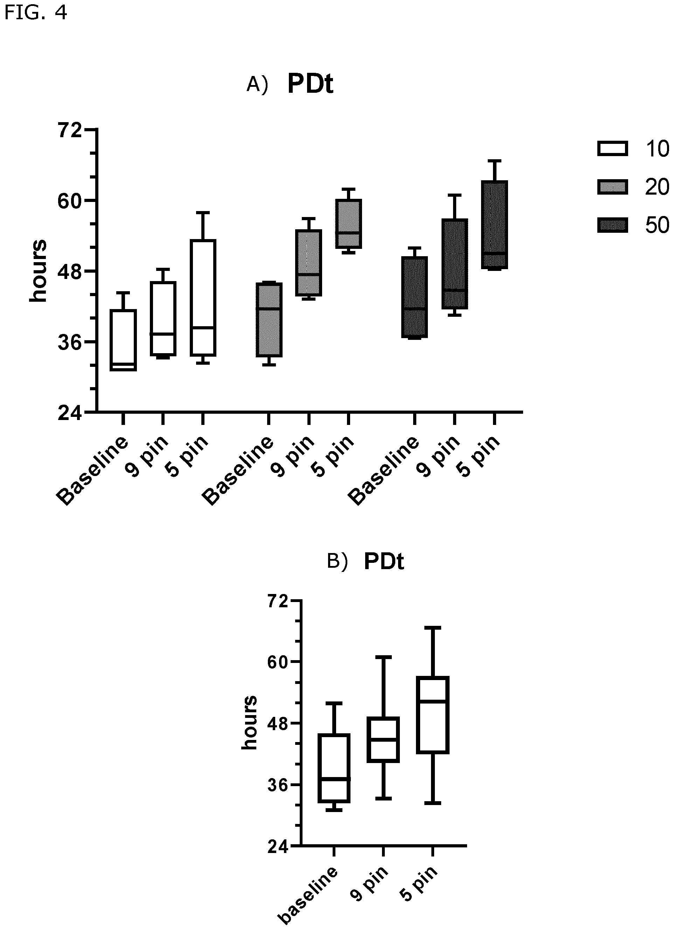

**Figure 4.** The observation in Fig. 3 was confirmed when assessing the population doubling time (PDt), as a significant prolonged PDt was seen when cells were ejected through 9-pin or 5-pin head compared with baseline (p<0.0001 and p=0.0063, respectively), with 9 pin being the least evasive (A) (B). See also Table 2.

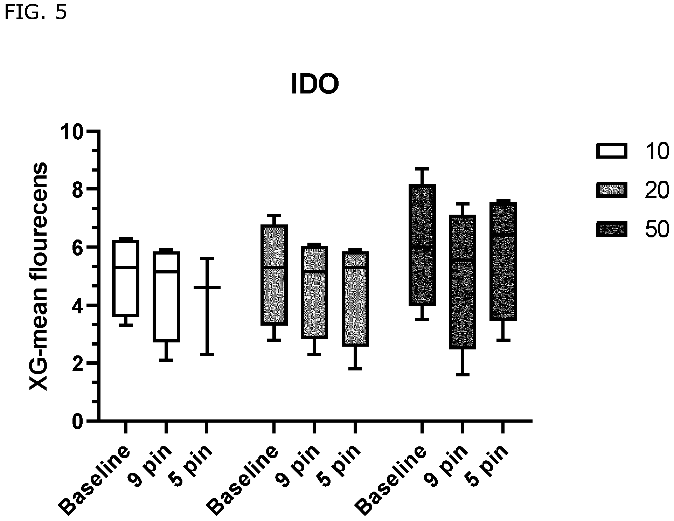

**Figure 5.** Administration through the needles did not affect the investigated ASCs immunomodulatory potency as there was no significant difference in IDOI expression, regardless of either cell concentration (10, 20 or 50 x10$^6$ ASCs/ml) or type of multi-needle head used (9- or 5-pin).

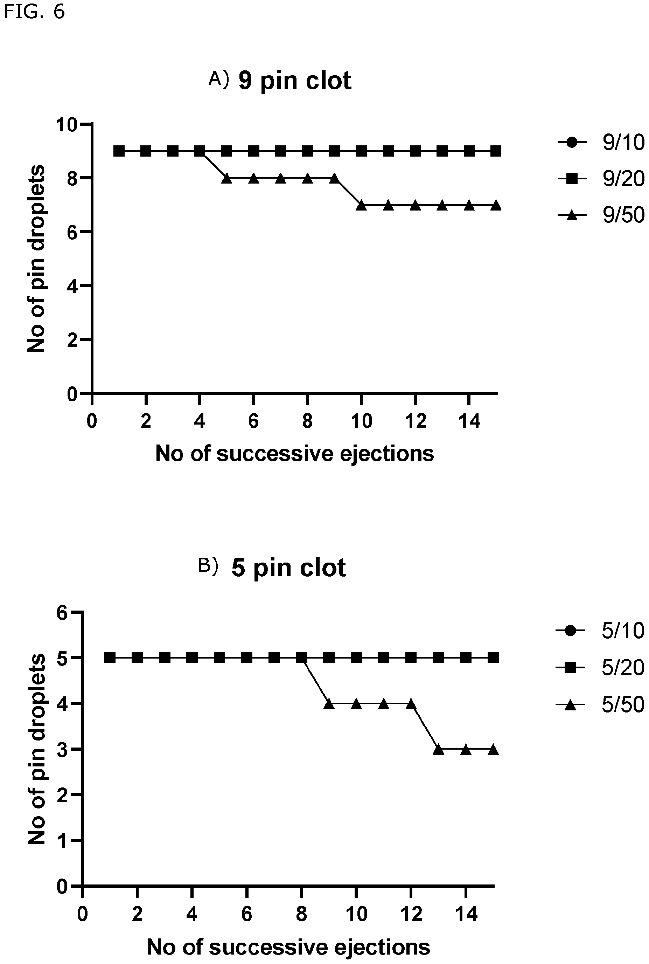

**Figure 6.** To evaluate the needle clogging tendency, ASC suspensions of 10, 20 and 50 x10$^6$ cells/ml) were consecutively ejected through either the 9-pin (A) or the 5-pin (B) head and the number of droplets counted. While the 10 and 20 x10$^6$ cells/ml suspensions demonstrated no clog tendency, the suspension of 50 x10$^6$ cells/ml showed a propensity to clog the needles of both 9 and 5 pins when the same multi-needle head was used for repeated ejections (first clog after the 4$^{th}$ and 8$^{th}$ successive ejection, respectively).

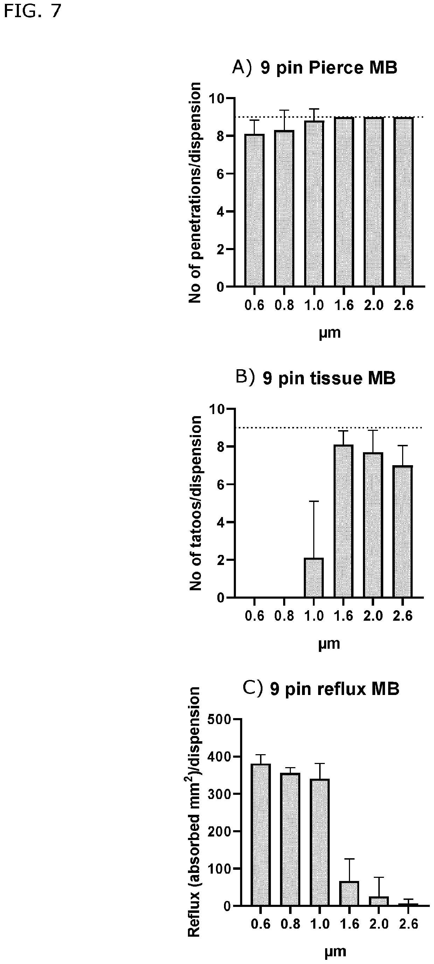

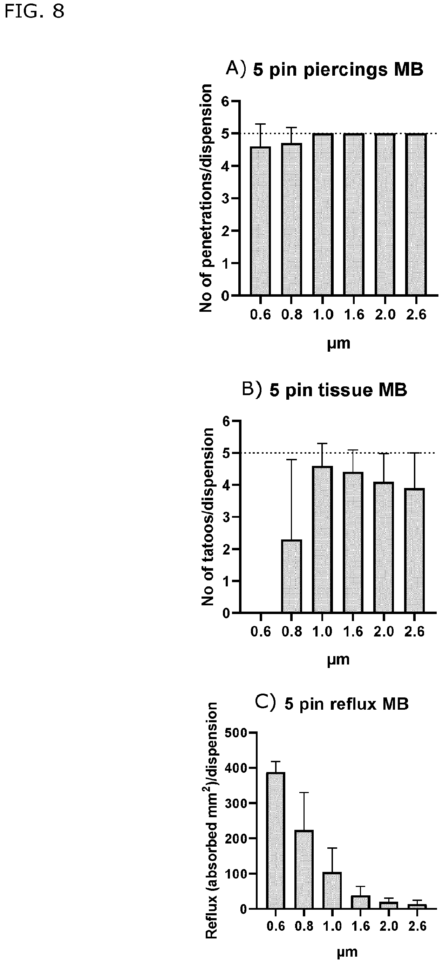

**Figure 7.** To validate successful cutaneous delivery of gradually deeper injections, using a 9-pin head, Methylene Blue (MB) solution was used as trace dye to assess skin penetration, dermal deposition and reflux in human skin, as described in the Materials and Methods section of Example 1. Using an injection depth above 1.0 mm (1.6-2.6 mm) resulted in all needles piercing the skin (PE 100%) (A), with dermal deposition from 7-8 needles (B) with minimal reflux to the skin surface (C). Similar good results were seen with the 5-pin head, though already from a depth of 1 mm as shown in Fig. 8.

**Figure 8.** To validate successful cutaneous delivery of gradually deeper injections, using a 5-pin head, Methylene Blue (MB) solution was used as trace dye to assess skin penetration, dermal deposition and reflux in human skin, as described in the Materials and Methods section in Example 1. Using an injection depth already above from 1.0 mm resulted in all needles piercing the skin (PE 100%) (A), with dermal deposition from more than 4 needles (B) with minimal reflux to the skin surface (C).

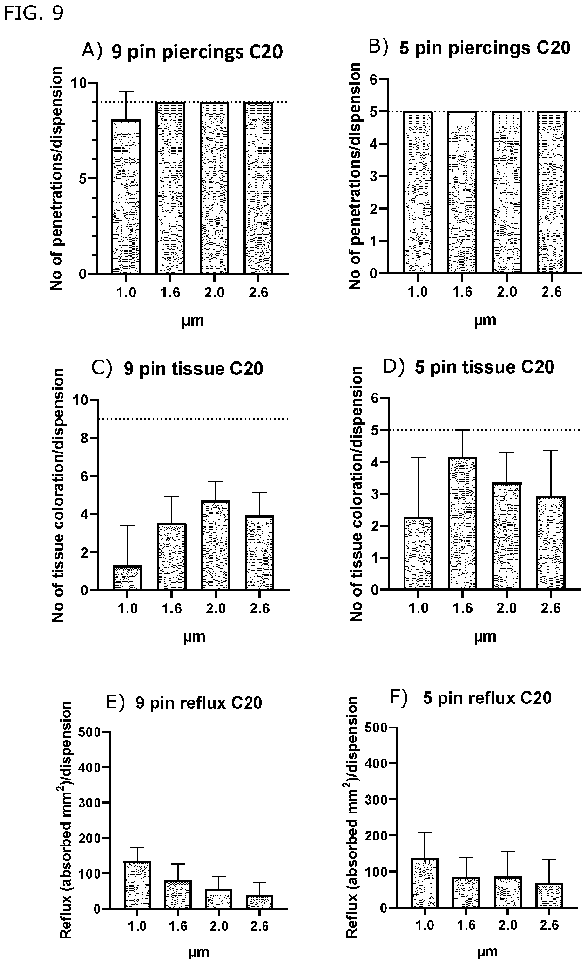

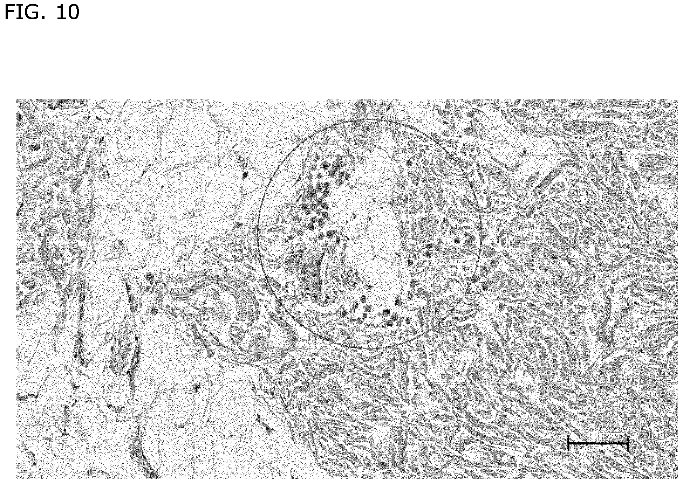

**Figure 9.** To evaluate the rate of successful skin penetration and delivery of cells in the target tissue, skin piercing with the 9- and 5-pin heads with 20 × 10$^6$ cells/ml was assessed ((A) and (B) for 9- and 5-pin head, respectively) along with reflux ((C) and (D) for 9- and 5-pin head, respectively). Needle skin piercings were observed for all depths. Increasing dermal deposition (tissue coloration) and decreased reflux of injected cell solution were observed with increased target depth ((E) and (F) for 9- and 5-pin, respectively). While coloration of target tissue was preliminarily evaluated, the results were not deemed optimally quantitative since the staining of the cell suspension was too weak for adequate sensitivity when macroscopically examined. However, the efficiency of dermal deposition using a suspension of 20 × 10$^6$ cells/ml can be expected to be as good as injecting MB (with similar viscosity), which is supported by data from the clogging assay (Fig. 6) where no needle clog tendency was observed, and as such allows undisturbed and, consequently, successful flow deposits into the dermis. Successful dermal cell deposition was ultimately confirmed by histological evaluation (Fig. 10) and by high frequency skin ultrasound imaging, as described in Example 1.

**Figure 10.** Histological evaluation (H&E) of skin tissue intradermally injected with ASCs (20 x $10^6$ cells/ml) confirmed the presence of injected ASCs within the dermis. It showed sites of closely packed cells within the target site, i.e the dermis (circle) with normal surrounding dermal fibrous tissue.

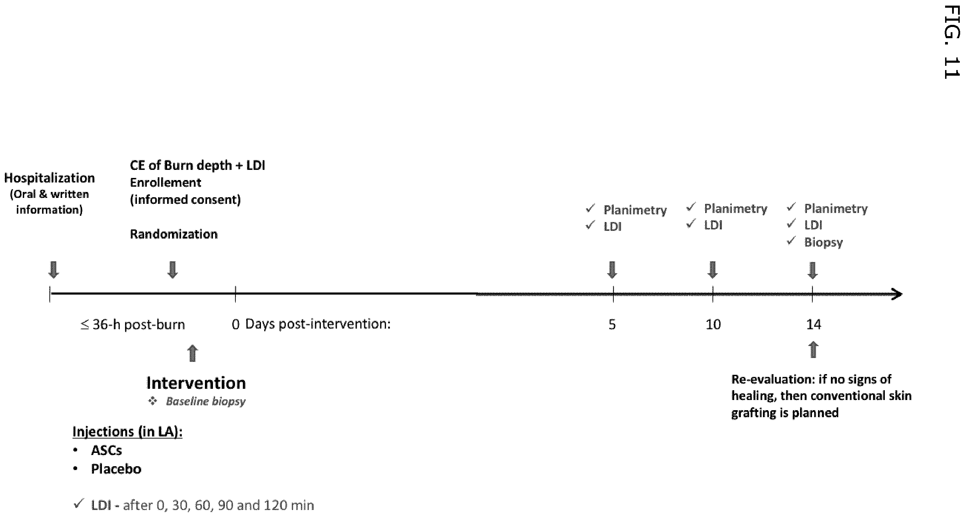

**Figure 11.** Timeline for the clinical study described in Example 2. Clinical Evaluation (CE), Local Anaesthesia (LA) with topical anaesthetic Lidocaine-prilocaine 5% cream (Emla ™), Adipose-derived Stem cells (ASCs), Laser Doppler Imaging (LDI).

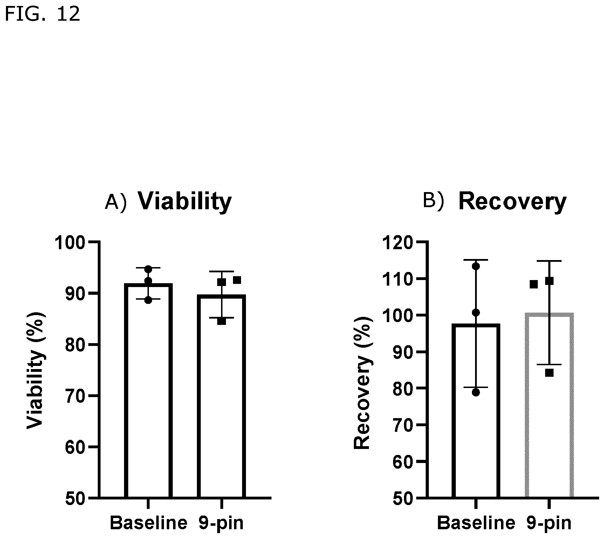

**Figure 12.** Baseline and cells ejected through a 9-pin head showed similar high perceived viability (percent viable cells) of approx. 90% (A). Recovery of viable cells (number of viable cells relative to the cell number frozen) was also high, actually more than 100% was recovered in most samples (B). The higher than 100% recovery indicate that more than the expected 22 x$10^6$ cells/ml might have been cryopreserved. Importantly, perceived viability and recovery was not significantly different when comparing baseline to cells ejected through 9-pin head. Also, these results are comparable to what was achieved with samples cryopreserved with CryoStor CS5 (5% DMSO), described in Example 1 (i.e. Fig. 1).

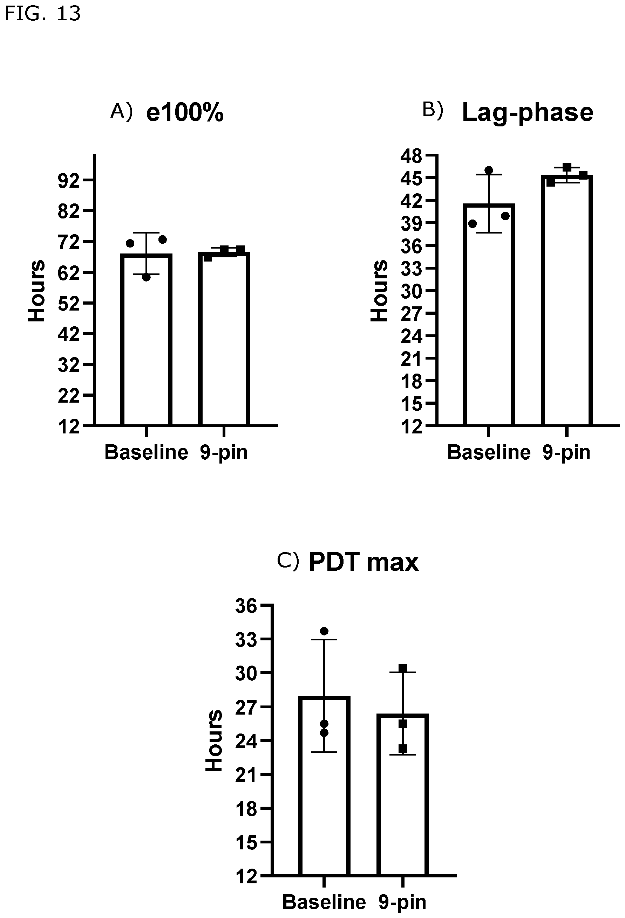

**Figure 13.** This figure shows that the proliferative potential of the cells seemed to be intact when ejected through the 9-pin head, compared to baseline. (A) e100%: Time to 100% cell confluency. (B) Lag-phase: Time from seeding to acceleration of cell division. (C) PDt max: Fastest population doubling time during culture.

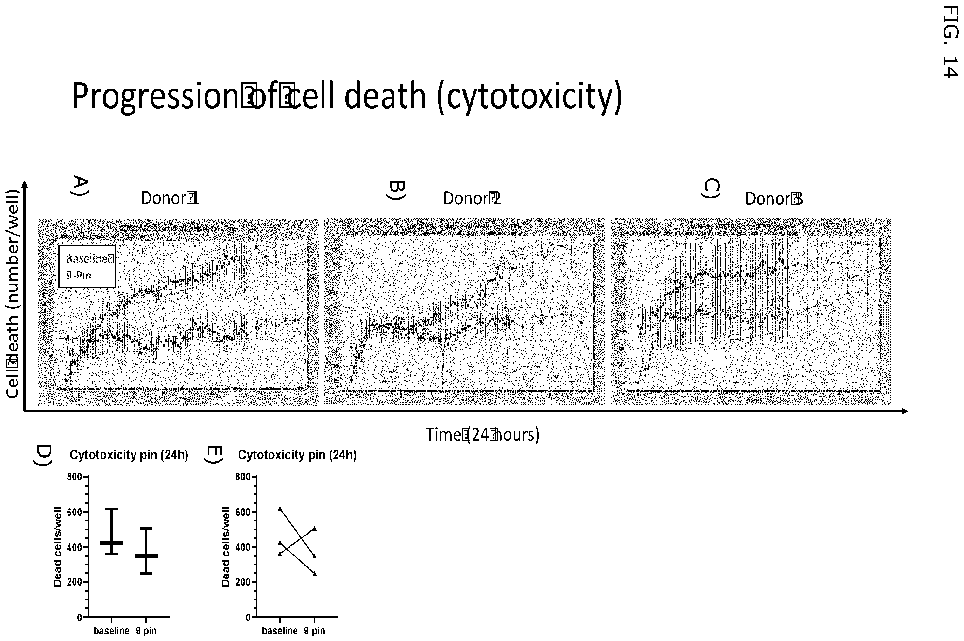

**Figure 14.** Long-time cell viability (cytotoxicity). Cells with an intact membrane may prove non-viable over time. The cytotoxicity assay used detected accumulated cell death over 24 hours. The results showed an initial progression of cell death within the first couple of hours, which seemed to decline ((A) to (C) for Donors 1-3, respectively). Results do not indicate that cells are damaged and subsequently die, when ejected through 9-pin, compared to baseline ((D) and (E)). See Example 3 for details.

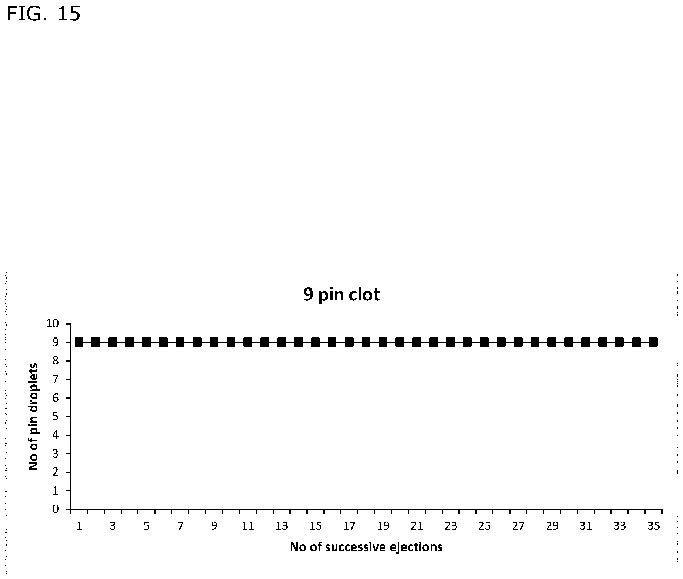

**Figure 15.** To evaluate the needle clogging tendency, the study drug containing cryopreserved suspension of 22 million ASCs/mL in Cryostor® CS10 was consecutively ejected through the 9-pin head and the number of droplets counted. The 22 x$10^6$ cells/ml suspension demonstrated no clog tendency when the same multi-needle head was used for repeated ejections, even up to 35 consecutive ejections.

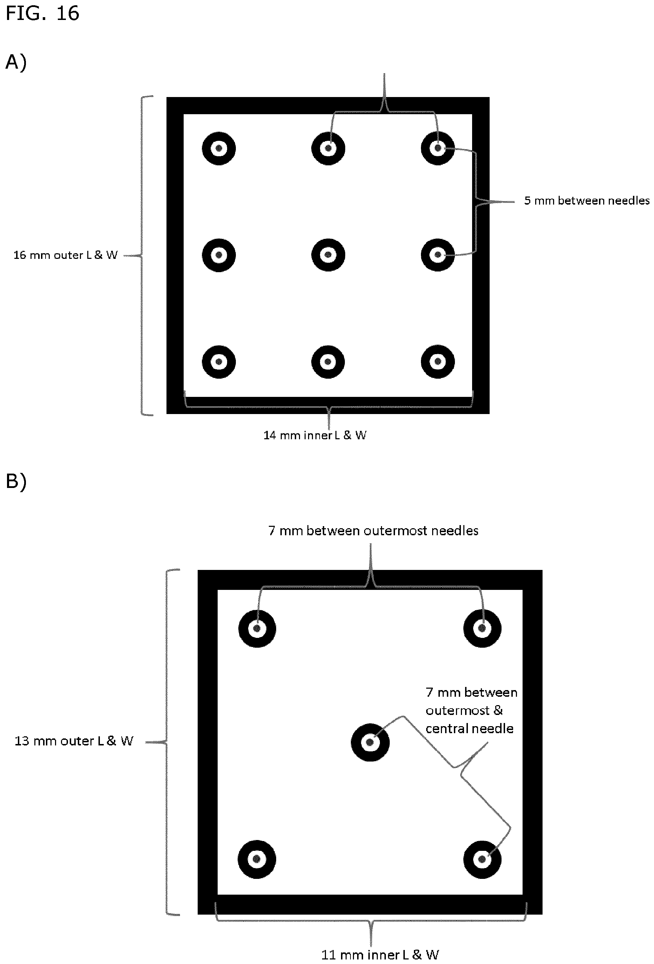

**Figure 16.** Dimensions of exemplary 9- & 5-pin needle head footprint for injection device (see Example 1).

DETAILED DISCLOSURE OF THE INVENTION

*Definitions*

As used herein, the following terms and phrases shall have the meanings set forth below. Unless defined otherwise, all technical and scientific terms used herein have the same meaning as commonly understood to one of ordinary skill in the art to which this invention belongs.

As used herein, "stem cells" are cells with the ability to divide for indefinite periods in culture and give rise to specialized cells. The stem cells may be unipotent, bipotent, or multipotent, having the ability to differentiate into one or more cell types, which perform one or more specific functions and which have limited or no ability to self-renew. Some stem cells contemplated herein may be pluripotent. Non-limiting examples of types of stem cells include somatic (adult) stem cells, embryonic stem cells, parthenogenetic stem cells and induced pluripotent stem cells (iPS cells or iPSCs). Somatic stem cells, also known as adult stem cells, are undifferentiated cells found in a differentiated tissue that can renew themselves (clonally) and (with certain limitations) differentiate to yield the specialized cell types of the tissue from which it originated. Non-limiting examples of adult stem cells include mesenchymal stem cells, hematopoietic stem cells, mammary stem cells, intestinal stem cells, endothelial stem cells, neural stem cells, olfactory adult stem cells and neural crest stem cells. Embryonic stem cells are primitive (undifferentiated) cells from an embryo that have the potential to become a wide variety of specialized cell types. Non-limiting examples of embryonic stem cells include the cell-lines eligible for use in NIH funded research available at grants.nih.gov/stem cells/registry/current.htm (last accessed 24 April 2020). Pluripotent embryonic stem cells can be distinguished from other types of cells by the use of markers including, but not limited to, Oct4, alkaline phosphatase, CD30, TDGF-1, GCTM-2, Genesis, Germ cell nuclear factor, SSEA1, SSEA3, and SSEA4. Induced pluripotent stem cell (iPSC) are artificially derived stem cells from non-pluripotent cells, typically adult somatic cells, produced by inducing expression of one or more stem cell specific genes. The characterization of stem cells and their use in regenerative medicine and disease therapeutics has been reviewed by, e.g., Mahla RS (2016).

As used herein, "mesenchymal stem cells" (MSCs) or "mesenchymal stromal/stem cells" refers to multipotent stromal stem cells which can differentiate into a variety of cell types, including one, more or all of osteoblasts (bone cells), chondrocytes (cartilage cells), and adipocytes (fat cells which give rise to marrow adipose tissue) under appropriate conditions. MSCs may be characterized by their tissue of origin, and are derivable from, e.g., adipose tissue, bone marrow, umbilical cord tissue (typically Wharton's jelly), umbilical cord blood, dental pulp and amniotic fluid. In some embodiments, MSCs are also plastic-adherent when maintained in standard culture conditions and express CD105, CD73 and CD90. In particular embodiments, MSCs are further characterized by a lack of expression of CD45, and optionally of CD34, CD14 or CD11b, CD79alpha or CD19 and HLA-DR surface molecules.

"Derivable from", "derived from" or the like as used herein in relation to stem cells derived/derivable from a particular tissue or organ refers to stem cells isolated from the tissue or organ in question. Typically, the isolation also comprises one or more subsequent purification and/or cultivation steps to increase the homogeneity of the stem cell preparation.

While a stem cell preparation or suspension derived from a tissue may, at least in some embodiments, contain some other cells from that tissue, the compositions for use in the therapeutic methods described herein typically comprise a stem cell population which is substantially homogenous, meaning that the majority of the cells comply with the standards defining that stem cell population.

As used herein, "adipose tissue-derived mesenchymal stem cells", "adipose tissue-derived stem cells," "adipose tissue-derived stromal cells" and the like, refer to MSCs which are derived from adipose tissue, and are herein referred to as "ASCs". Certain criteria for identifying ASCs are known in the art and are described in, for example, Bourin et al. (Cytotherapy. 2013 June; 15(6): 641-648), which is incorporated by reference in its entirety. In some embodiments, ASCs are characterized by their ability to differentiate along adipocytic, chondroblastic and osteoblastic lineages under appropriate conditions. ASCs in culture may be characterized by expression of one or more of the following cell-surface markers: CD90, CD73, CD105 and lack of expression of CD45 and CD31. In some embodiments, they can be distinguished from bone marrow-derived MSCs by their positivity for CD36 and negativity for CD106.

By "adipose" is meant any fat tissue. The adipose tissue may be brown or white adipose tissue, derived from the abdominal area or other adipose tissue site. In certain embodiments the adipose is subcutaneous white adipose tissue or visceral adipose tissue or any other tissue containing adipose cells. Preferably, the adipose tissue is human, most preferably from an adult human. A convenient source of adipose tissue is from liposuction surgery.

As used herein, "skin lesion" means any skin damage, rupture or abnormality caused by an injury, a disorder or a disease, typically involving at least the epidermis and part or all of the dermis in the affected skin area. "An injury" may also be referred to herein as a "physical injury" or "traumatic injury", and include thermal, electrical, chemical, radiation and/or physical/mechanical injuries. Non-limiting disorders and diseases known to cause skin lesions include inflammatory, autoimmune, allergic, hypersensitivity, degenerative, ischemic, rheumatic, metabolic, endocrine, and benign neoplastic diseases and disorders. Non-limiting examples of skin lesions include wounds, scars, ulcers and rashes. A morphological description of various types of skin lesions can be found at www.msdmanuals.com/professional/dermatologic-disorders/approach-to-the-dermatologic-patient/description-of-skin-lesions.

As used herein, a "wound" means a break in the continuity of the skin, involving loss of epidermis and typically also at least part of the underlying dermis. Wounds may be caused by external agents or influences (e.g., an outside force) or internal agents, etiologies, or pathologies and are typically classified mainly on the basis of mode of infliction and causative agent. For example, wounds caused by external agents and influences include, without limitation, mechanical wounds and burn wounds. A burn wound may be caused by, e.g., a thermal, electrical, chemical, radiation and/or friction injury. A detailed classification of the burn depth of burn wounds can be found in Table 3. However, burn wounds are dynamic and often progress in depth over time, which means that the classification of a particular burn wound may be different at different times after the burn injury, i.e., at different times "post-burn". Wounds caused by internal agents, etiologies or pathologies, which may be referred to as "ulcers", include, without limitation, diabetic wounds/ulcers, venous wound/ulcers, ischemic wounds/ulcers, neoplastic wounds/ulcers, and vasculitic and autoimmune wounds/ulcers. Wounds may also be referred to as "acute" or "chronic" depending on the length of time that the wound has existed, where "chronic" wounds do not heal in an orderly set of stages and in a predicable amount of time, e.g., within three months.

As used herein, a "scar" is an area of fibrous tissue that replaces normal skin after an injury or trauma. Non-limiting types of scars include, for example, keloid scars and hypertrophic scars. Scars resulting from deep burn wounds healed by secondary healing, or sometimes even following conventional surgical treatment with skin grafting, are often characterized by thick hypertrophic scarring and scar contractures, substantially restricting movement of joints, which may be cosmetically, functionally and psychosocially debilitating and detrimental for the patient.

"Debridement" is the medical removal of dead, damaged, or infected tissue to improve the healing potential of the remaining healthy tissue. "Necrectomy", as used herein, means the surgical or "sharp" removal of necrotic tissue, e.g., eschar or slough, using a surgical tool such as a scalpel or scissors or other sharp bladed instrument, with "eschar" referring to non-viable, necrotic tissue caused by, for example, a burn of full thickness. As used herein, "escharectomy" is the surgical or "sharp" debridement of eschar. Escharectomy or necrectomy is the standard treatment for full thickness burn injuries, and involves surgical excision down to the level of tissue that exhibits punctate bleeding, i.e., clear, visual evidence that the excision has reached a viable tissue bed. The procedure is usually performed before closing the wound with autologous skin grafts.

The term "cryopreserve", "cryostore" or its various grammatical forms as used herein refers to preserving cells for storage in a cryoprotectant at sub-zero temperatures. For long-term storage, cryovials containing the cells and cryoprotectant are usually placed in liquid nitrogen.

The term "cryoprotectant" as used herein refers to an agent that minimizes ice crystal formation in a cell or tissue, when the cell or tissue is cooled to sub-zero temperatures and results in substantially less damage to the cell or tissue after thawing in comparison to the effect of cooling without cryoprotectant.

A preparation of human ASCs "free of non-human animal proteins" means that the ASCs were produced by a process where they did not come into contact with proteins derived from non-human animals.

"Viability" as used herein refers to the feature of cells of not taking up membrane impermeant dye (e.g., Trypan Blue, Cytotox red, FVS-780, SYTOX blue, propidium iodide, acridine orange, DAPI (4',6-diamidino-2-phenylindole), 7AAD (7-amino actinomycin D) etc.), thereby demonstrating cell membrane integrity.

"Proliferative capacity" as used herein refers to the ability of cells to multiply in a suitable cultivation medium. Proliferative capacity can, for example, be represented by the relative number of cells after a 24h, 48h or 72h cultivation period as compared to the number of cells initially plated. This can also be expressed as "population doublings" during a certain period. For example, a population doubling of at least 1 during 48h in cell culture means that the number of cells seeded have doubled at least once during that period. The population doubling time or "PDT" of a cell population can, for example, be identified by determining the growth rate of a cell population using automated analysis (e.g., the IncuCyte® Live-Cell Analysis Systems (Essen Bioscience)) and then calculating the PDT (in hours) using Formula I shown in Example 1.

"Adhesion" or "spreading" as used herein refers to the ability of cells to adhere to a surface (e.g., a plastic tissue culture well), thereby loosing its initial rounded shape. Adhesion/spreading ability can be evaluated by determining the eccentricity value of a cell population (using, e.g., automated analysis techniques such as those provided by the IncuCyte® Live-Cell Analysis Systems (Essen Bioscience)), with a low eccentricity value representing a rounded shape and the fraction of cells having a high eccentricity value (e.g., an eccentricity value of at least 0.8 representing cell adhesion/spreading).

The terms "treatment," "therapy" and the like are used herein to generally refer to obtaining a desired pharmacological, physiological and/or biological effect. Unless contradicted by context, the effect may be prophylactic in terms of completely or partially preventing a disease or disorder, or a pathological process or symptom associated with the disease or disorder, and/or may be therapeutic in terms of a partial or complete stabilization or cure for a disease or disorder and/or an adverse effect or complication attributable to the disease or disorder. For example, in the case of a burn wound, since a burn wound is a dynamic injury that progresses in time, preventing further progression/tissue damage or preventing a burn wound from becoming a considerable scar can also be encompassed by the term "treatment" of a burn wound. "Treatment" as used herein covers any treatment of a disease or disorder in a mammal, particularly a human, and includes: (a) preventing the disease or disorder or an associated symptom or complication from occurring in a subject which may be predisposed to the disease or disorder or the symptom or complication but has not yet been diagnosed as having it; (b) inhibiting a disease or disorder or a symptom or complication, *i.e.,* arresting its development; or (c) relieving a disease or disorder or a symptom or complication of a disease or disorder, *i.e.,* causing regression of the disease, disorder, symptom and/or complication. For example, in the case of a burn wound or other type of wound, an accelerated degree and/or rate of healing of the wound can also be encompassed by the term "treatment." Additional parameters that may represent treatment of a burn wound or other wound include, but are not limited to, (a) a reduced extent of tissue damage or necrosis of the wound; (b) a reduced extent of apoptosis in the wound; (c) a reduced extent of local inflammation; (d) an increased revascularisation and/or angiogenesis of the wound; (e) an increased blood perfusion of the wound; (f) an increased and/or accelerated regeneration of skin appendages and normal anatomical structures of the skin of the wound; (g) an increased degree and/or rate of healing of the wound; (h) a reduced need for later skin grafting; (i) a reduced extent of scarring and/or fibrosis of the wound; and (j) an improved quality of the scar from the wound.

As used herein, the term "pharmaceutical composition" refers to a composition intended for use in therapy of a human patient. A pharmaceutical composition according to the present disclosure typically comprises stem cells, preferably MSCs, most preferably ACSs. However, the pharmaceutical composition may additionally include other pharmaceutically acceptable, non-cellular components, such as pharmaceutically acceptable carriers.

The phrase "pharmaceutically acceptable" is employed herein to refer to compounds, materials, compositions, and/or dosage forms which are, within the scope of sound medical knowledge and judgment by a person of skill in the art, suitable for use in contact with the tissues of human beings without excessive toxicity, irritation, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, or excipient, including but limited to viscous liquids such as gel formulations, which are known in the art.

As used herein, a "therapeutically effective amount" refers to the amount of an active agent (e.g., MSCs, ASCs) sufficient to induce a desired biological result, such as a prevention, delay, reduction or inhibition of a skin lesion or one or more symptoms of a given skin disease or disorder. A "therapeutically effective amount" as used herein may also denote an amount of the active agent (e.g., MSCs, ASCs) causing a measurable improvement of a skin lesion or in one or more symptoms. The amount may vary with the lesion or condition being treated, the stage of advancement of the condition, and the type and concentration of active agent applied. Appropriate amounts in any given instance will be readily apparent to those skilled in the art, such as a physician, or capable of determination by routine experimentation, particularly based on the present disclosure.

The terms "recipient", "subject" and "patient" are used interchangeably herein and refer to the subject for whom treatment or therapy is desired, particularly a human subject.

In the context of therapeutic use of the disclosed pharmaceutical compositions, in "allogeneic" therapy, the donor and the recipient are genetically different individuals of the same species, whereas in "autologous" therapy, the donor and the recipient is the same individual.

As used herein, the term "donor" refers to the human from which stem cells have been retrieved. Preferably, the human is an adult.

In the context of the present invention, unless contradicted by context, "about", "approximately" or the like, typically refers to a variation (+/-) of at most 20%, such as at most 10%, such as at most 5%, from the reference value. So, for example, about $2.0 \times 10^8$ cells may include or correspond to from $1.6 \times 10^8$ to $2.4 \times 10^8$ cells, such as from $1.8 \times 10^8$ to $2.2 \times 10^8$ cells, such as from $1.9 \times 10^8$ to $2.1 \times 10^8$ cells.

*Specific aspects and embodiments of the invention*

The following aspects are particularly contemplated:
A composition comprising a suspension of human stem cells for use in the treatment of a skin lesion in a human subject, comprising administering the composition to the skin lesion by intradermal or transdermal injection using one or more injection needles inserted into the skin at an angle of at least about 60 degrees to the skin surface.

A composition comprising a suspension of human stem cells for use in the prevention of a skin lesion in a human subject, comprising administering the composition to a skin area at risk for a skin lesion by intradermal or transdermal injection using one or more injection needles inserted into the skin at an angle of at least about 60 degrees to the skin surface.

A composition comprising allogeneic ASCs for use in treating a burn wound in the skin of in a human subject, comprising administration of from about $1 \times 10^4$ to about $1 \times 10^6$ ASCs per $cm^2$ of a burn wound, without prior escharectomy or necrectomy.

A method of treating a skin lesion in a subject in need thereof, the method comprising administering an effective amount of a composition comprising a suspension of stem cells to the skin lesion by intradermal or transdermal injection using one or more injection needles inserted into the skin at an angle of at least about 60 degrees to the skin surface.

A method of preventing a skin lesion in a subject in need thereof, the method comprising administering an effective amount of a composition comprising a suspension of stem cells to a skin area at risk for a skin lesion by intradermal or transdermal injection using one or more injection needles inserted into the skin at an angle of at least about 60 degrees to the skin surface.

A method of treating a skin disease or disorder in a subject in need thereof, the method comprising administering an effective amount of a composition comprising a suspension of stem cells to a skin lesion associated with the skin disease or disorder by intradermal or transdermal injection using one or more injection needles inserted into the skin at an angle of at least about 60 degrees to the skin surface.

A method of treating a skin disease or disorder in a subject in need thereof, the method comprising administering an effective amount of a composition comprising a suspension of stem cells to a skin area at risk for a skin lesion associated with the skin disease or disorder by intradermal or transdermal injection using one or more injection needles inserted into the skin at an angle of at least about 60 degrees to the skin surface.

A method of administering a composition comprising stem cells into the dermis or hypodermis of a subject, the method comprising administering a suspension of stem cells to a predetermined skin area by intradermal or transdermal injection using one or more injection needles inserted into the skin at an angle of at least about 60 degrees to the skin surface.

A method of treating a burn wound in a subject in need thereof, the method comprising administering an effective amount of a composition comprising a suspension of stem cells to a burn wound by intradermal or transdermal injection using one or more injection needles inserted into the skin at an angle of at least about 60 degrees to the skin surface.

A method of reducing the extent of a tissue damage or necrosis of a burn wound, reducing the extent of apoptosis in a burn wound, reducing the extent of local inflammation in a burn wound, increasing the revascularisation of a burn wound, increasing the blood perfusion of a burn wound, increasing the regeneration of skin appendages and normal anatomical structures of the skin of the burn wound, increasing the degree and/or rate of healing of a burn wound, reducing the need for later skin grafting of a burn wound, reducing the extent of scarring/fibrosis of a burn wound and/or improving the quality of the scar from a burn wound, the method comprising administering an effective amount of a composition comprising a suspension of stem cells to a burn wound by intradermal or transdermal injection using one or more injection needles inserted into the skin at an angle of at least about 60 degrees to the skin surface.

A method of treating a burn wound in the skin of in a human subject, the method comprising administering a composition comprising allogeneic ASCs at a dosage of from about $1 \times 10^4$ to about $1 \times 10^6$ ASCs per $cm^2$ of the burn wound, without prior escharectomy or necrectomy of the burn wound.

A method of reducing scar formation from a burn wound in a subject in need thereof, the method comprising administering a composition comprising allogeneic ASCs at a dosage of from about $1 \times 10^4$ to about $1 \times 10^6$ ASCs per $cm^2$ of the burn wound, without prior escharectomy or necrectomy of the burn wound.

A method of preventing the progression of a partial-thickness burn wound into a deep partial-thickness burn wound and/or a full-thickness burn wound, the method comprising administering a composition comprising allogeneic ASCs at a dosage of from about $1 \times 10^4$ to about $1 \times 10^6$ ASCs per $cm^2$ of the burn wound, without prior escharectomy or necrectomy of the burn wound.

In one particular embodiment of any one of these aspects, the composition comprises from about $1 \times 10^6$ per mL to about $1 \times 10^8$ per mL ASCs or other MSCs and is administered by intradermal injection at between 3 and 18 insertion sites per $cm^2$ of skin using a delivery device,
wherein the delivery device is adapted to deliver a liquid formulation through a plurality of substantially parallel injection needles when inserted into the skin and to keep the skin area in and around the insertion site of each injection needle taut during at least the needle insertion and injection phases and the injection needles have an inner diameter of at least 0.1 mm and a length of no more than about 3 cm, and
wherein the composition is administered by a method comprising the steps of

(b) applying the device to a predetermined area of the skin, wherein the device is loaded with a volume of the composition and activated to create a vacuum, suction or mechanical force to gently lift the skin towards a plurality of surfaces, each surface comprising an opening from which an injection needle can emerge;

(c) activating the device to

i. insert the tip of each injection needle into the skin at an angle of at least about 80 degrees to the skin surface to an injection needle insertion/injection depth of at least about 0.6 mm; and

ii. inject from each injection needle about 1 μL to about 10 μL of the composition at an injection rate of no more than about 0.4 mL/min, wherein, after the injection phase, each injection needle remains inserted into the skin for a period of at least 3 seconds before retraction;

(d) optionally, repeating steps (a) and (b) at an adjacent or other predetermined area of the skin.

In one particular embodiment of any one of these aspects, the composition comprises from about $1 \times 10^7$ per mL to about $4 \times 10^7$ per mL ASCs or other MSCs and is administered by intradermal injection at between 7 and 11 insertion sites per $cm^2$ of skin using a delivery device,
wherein the delivery device is adapted to deliver a liquid formulation through a plurality of substantially parallel injection needles when inserted into the skin and to keep the skin area in and around the insertion site of each injection needle taut during at least the needle insertion and injection phases and the injection needles have an inner diameter of from about 0.13 mm to about 0.26 mm and a length of no more than about 2 cm, and
wherein the composition is administered by a method comprising the steps of

(a) applying the device to a predetermined area of the skin, wherein the device is loaded with a volume of the composition and activated to create a vacuum, suction or mechanical force to gently lift the skin towards a plurality of surfaces, each surface comprising an opening from which an injection needle can emerge;

(b) activating the device to

i. insert the tip of each injection needle into the skin at an angle of at least about 85 degrees to the skin surface to an injection needle insertion/injection depth of at least about 1.5 mm; and

ii. inject from each injection needle about 2 µL to about 5 µL of the composition at an injection rate of no more than about 0.22 mL/min, wherein each injection needle remains inserted into the skin for a period of at least 6 seconds before retraction;

(c) optionally, repeating steps (a) and (b) at an adjacent or other predetermined area of the skin.

In some embodiments, the plurality of substantially parallel injection needles is between 2 to 27, such as between 3 and 18, such as between 5 and 15, such as between 7 and 11, such as 5 or 9.

Compositions

The composition for the use according to the aspects and embodiments described herein comprise human stem cells, including, but not limited to somatic (adult) stem cells, embryonic stem cells, parthenogenetic stem cells and induced pluripotent stem cells (iPS cells or iPSCs). In some embodiments, the somatic (adult) stem cells are selected from mesenchymal stem cells (MSCs), hematopoietic stem cells, mammary stem cells, intestinal stem cells, endothelial stem cells, neural stem cells, olfactory adult stem cells and neural crest stem cells.

In some embodiments, the human stem cells are stem cells derived from skin. For example, the preparation of human stem cells from skin have been described in, e.g., Toma et al. Nat Cell Biol. 2001;3(9):778-84; Nowak et al. Methods Mol Biol. 2009;482:215-32; and US Patent Application Publication No. 2007/0248574.

In some embodiments, the human stem cells are derived from eschar. The preparation of stem cells from eschar has been described in, e.g., Van der Veen, et al. (2012), Cell Transplant. 21(5):933-42 and WO2016054592A1.

Preferably, the human stem cells are MSCs. The characteristics of MSCs can promote a fast and effective healing of wounds and other skin lesions, such as skin lesions caused by ischemic, inflammatory or autoimmune diseases and disorders. Moreover, in particular for treatment of burn wounds, MSCs may be useful in terms of reducing inflammation, burn progression, and accelerating healing of the skin with restored barrier function and reduced scarring. MSCs for use according to the aspects and embodiments described herein can be derived from nearly all body tissues. Preferably, however, MSCs for use in the aspect or embodiments described herein are derived from adipose tissue, bone marrow, umbilical cord tissue (e.g., Wharton's jelly), umbilical cord blood, dental pulp or amniotic fluid; more preferably from adipose tissue, bone marrow, umbilical cord tissue or umbilical cord blood. Methods of preparing MSCs from various types of tissues are known in the art.

Exemplary, non-limiting methods for isolation of stem cells from bone marrow useful in the aspects and embodiments disclosed herein are described in, e.g., Gnecchi and Melo, Methods Mol Biol 2009;482:281-94 and WO2011047289A1.

Exemplary, non-limiting methods for isolation of stem cells from regenerative cells from dental pulp useful in the embodiments disclosed herein are described in, e.g., U.S. 2012/0251504 and Noda et al., Sci Rep 2019;9:5430.

Exemplary, non-limiting methods for isolation of stem cells from Wharton's jelly useful in the embodiments disclosed herein are described in, e.g., U.S. Patent Application Publication Nos. 2013/0183273 and 2011/0151556, WO 04/072273A1, Corotchi, et al. Stem Cell Research and Therapy 2013;4:81.

Exemplary, non-limiting methods for isolation of stem cells from amniotic fluid useful in the embodiments described herein are described in, e.g., U.S. Patent No. 8021876, International Patent Application Publication Nos. WO 2010/03369 A1, WO 2012/014247 A1 and WO 2009/052132, and U.S. Patent Application Publication Nos. 2013/0230924 and 2005/0054093.

Exemplary, non-limiting methods for isolation of stem cells from the umbilical cord useful in the embodiments described herein are described in, e.g., U.S. Patent Application Publication No. 20130065302, Reddy, et al. (2007), Methods Mol Biol. 407: 149-63, Hussain, et al. (2012) Cell Biol Int. 36(7):595-600, Pham, et al. (2014) Journal of Translational Medicine 2014, 12:56, Lee, et al. (2004) Blood 103(5): 1669-1675.

Furthermore, MSCs can be prepared from pluripotent stem cells, including embryonic stem cells and induced pluripotent stem cells (see, for example, Dayem et al., Int J Mol Sci. 2019 Apr; 20(8): 1922).

The compositions for use according to the aspects and embodiments described herein preferably comprise human allogeneic ASCs, *i.e.,* ASCs that are obtained or isolated from a healthy donor. Several different allogeneic ASC compositions proposed for use in therapy together with methods for preparing them have been described (see, e.g., WO 2017/068140; WO 2014/203267; WO 2017/144552 A1; Haack-Sorensen et al., Scand J Clin Lab Invest. 2018 Jul;78(4):293-300; Haack-Sørensen et al., J Transl Med. 2016 Nov 16;14(1):319; Panes et al., Lancet. 2016;388(10051): 1281-90) all of which are contemplated for use in the treatments described herein. However, autologous preparations of ASCs may also be used. These may be prepared according to known methods, e.g., as described in Lin et al., Cell Transplant. 2017 Mar; 26(3): 449-460.

While any suitable concentration of the stem cells, e.g., of MSCs such as ASCs, may be used in the compositions described herein, the concentration is typically at least $1 \times 10^6$ per mL, at most about $1 \times 10^8$ per mL or both. That is, in some embodiments, the concentration of stem cells, e.g., the MSCs such as ASCs, in the composition is in the range of from about $1 \times 10^6$ per mL to about $1 \times 10^8$. In some embodiments, the concentration of stem cells, e.g., the MSCs or ASCs, is at most about $5 \times 10^7$ per mL, such as at most about $4 \times 10^7$ per mL. In particular embodiments, the concentration of the stem cells, e.g., the MSCs such as ASCs, in the composition may be in the range of from about $1 \times 10^7$ to about $5 \times 10^7$ per mL, such as from about $1 \times 10^7$ to about $4 \times 10^7$ per mL.

Preferably, in a composition comprising ASCs or other MSCs, the concentration of the ASCs is at least about $1.5 \times 10^7$, such as at least about $2 \times 10^7$, such as at least about $3 \times 10^7$, such as at least about $4 \times 10^7$, such as at least about $5 \times 10^7$ ASCs or other MSCs per mL. In one embodiment, the concentration is from about $1.8 \times 10^7$ to about $2.6 \times 10^7$ ASCs per mL, such as from about $2.0 \times 10^7$ to about $2.4 \times 10^7$ ASCs per mL, such as about $2.2 \times 10^7$ ASCs per mL.

The ASCs or other MSCs suitable for the composition for the uses as described herein can be characterized by their multipotent capacity, marker profile, and/or and by functional characteristics, such as proliferation capacity, viability, recovery and immunosuppressive capability, even after cryopreservation and/or administration. Such characteristics are described in more detailed below. Suitable methods for determining these and other characteristics are described in WO 2017/068140 A1, which is hereby incorporated by reference in its entirety, as well as in the present Examples.

The ASCs and other MSCs are, in particular, characterized by their ability to differentiate along adipocytic, chondroblastic and osteoblastic lineages under appropriate conditions. For example, the ability of ASCs to differentiate along these lineages can be assessed by culture in differentiation medium according to the method described in Example 4 of WO 2017/068140 A1.

The ASCs and other MSCs can also or alternatively be characterized according to their phenotype, *i.e.*, marker profile, regarding their expression of markers, including CD90, CD73, CD105, and CD44, and maintaining low or negligible expression levels of CD45 and CD31 (Bourin *et al.*, 2013). Marker profiles can, for example, be conveniently determined by flow cytometry using fluorescence-labelled antibodies against each marker.

The MSC compositions for use in the therapeutic methods described herein preferably comprise an MSC population which is substantially homogenous, meaning that the majority of the cells comply with MSC standards for MSC derived from that tissue. For example, in one embodiment, the ASC compositions for use in the therapeutic methods described herein comprise an ASC population which is substantially homogenous, meaning that the majority of the cells comply with ASC standards.

Accordingly, in some embodiments, at least about 80% of the ASC population for use in an ASC composition express CD90, CD73, CD13, CD105, CD29, CD166, CD10, CD140b, CD160, CD204, CD272, CD44, CD49a, CD54, CD9, Galectin 3, Galectin 9, HLA-G and LTβR and at most about 15% of the ASC population express CD45, CD19, CD14, CD106, CD31 and CD36.

In some embodiments, of the ASC population for use in an ASC composition,

- at least 90% express CD90, CD73, CD13, CD29 and CD166; at most 5% express CD45, CD19, CD14 and CD31; at most 10% express CD106; between 2 and 15% express CD36; at least 10% express CD146; at least 80% express CD105 and at most 40% express CD34; and/or
- at least 90% express CD10, CD140b, CD160, CD204, CD272, CD44, CD54, CD9, Galectin 3, Galectin 9, HLA-G and LTβR; at least 80% express CD49a; at least 60% express CD258 and CD270 and at least 5% express CD200; at most 15% express CD15, CD152, CD163, CD18, CD274, CD39, CD40, CD62L, CD80 and CD86; and at most 30% express CXCR4.

The ASCs or other MSCs may also or alternatively be characterized by their anti-fibrotic properties. For example, the anti-fibrotic properties of an MSC population can be evaluated by contacting the MSC population or conditioned media from the MSCs with cultured fibroblasts, and then comparing its effect on the viability of the fibroblasts in comparison to a control. One example of a suitable assay is described in Yokoyama et al. (2008), where primary cardiac fibroblasts obtained from experimental rats (e.g., male Lewis rats) are plated onto 96-well plates ($4 \times 10^3$ cells per well), and, after 24h, changing the medium to conditioned medium from ASCs. After 48h, the cellular levels of 3-(4,5-dimethylthiazol-2-yl)-5-(3-carboxymethoxyphenyl)-2-(4-sulfophenyl)-2H-tetrazolium (MTS), indicative of the mitochondrial function and viability of the cardiac fibroblasts cells can be measured and compared to a control, e.g., cardiac fibroblasts incubated in non-conditioned control medium or conditioned medium from control cells, e.g., cardiac fibroblasts. The anti-fibrotic properties of an ASC population can also be determined by measuring the levels of anti-fibrotic factors, such as e.g. hepatocyte growth factor (HGF) and/or interleukin-10 (IL-10), secreted from the ASCs in comparison to a control, e.g., by ELISA (Yokoyama et al., 2008). Alternatively, the Scar-in-a-Jar model described by Chen et al. (Br J Pharmacol 2009;158(5): 1196-209) can be used, which is an *in vitro* assay studying the biosynthetic cascade of collagen matrix formation, including complete conversion of procollagen by C-proteinase/BMP-1, its subsequent extracellular deposition and lysyl oxidase-mediated cross-linking, achieved by applying the biophysical principle of macromolecular 'crowding'.

Other suitable assays include those described in Sharma et al., (Advanced Wound Care 2019;8(12);655) for evaluating anti-scarring drug targets in keloids.

The ASCs or other MSCs may further be characterized by angiogenetic properties. Suitable assays are known in the art, and include the ones described by, e.g., Lu et al. (Stem Cells International Volume 2018, Article ID 7537589), studying the effect of ASCs on endothelial colony-forming cells (ECFCs) *in vitro* and *in vivo,* and Pauti et al. (EBioMedicine. 2018 Jan;27:225-236), describing an *in vitro* model using a microvessel-on-a-chip, mimicking an effective endothelial sprouting angiogenesis.

The ASCs or other MSCs may also or alternatively be characterized by their immunosuppressive properties. For example, the ASCs or other MSCs may be characterized by one or more or all of the following: suppressing activation of dendritic cells (DCs), suppressing proliferation of peripheral blood mononuclear cells (PBMCs), cell surface markers indicative of immunomodulation, especially immunosuppression, or by a change in one or more cell surface markers in response to a cytokine such as interferon-gamma.

In one embodiment, the ASCs or other MSCs suppress activation of DCs, e.g., reducing the expression of CD40, CD80, CD86 and HLA-DR by DCs mixed with ASCs or other MSCs as compared to DCs not mixed with ASCs (*i.e.,* a positive control). In a specific embodiment, the assay of Example 9 of WO 2017/068140 A1 is used, wherein ASCs and DCs are seeded to result in approximately a 1:1 ratio; the DCs being stimulated with 1 μg/mL lipopolysaccharide (LPS) and 20 ng/mL interferon-gamma and incubated for 24 h; and the respective expression level of CD40, CD80, CD86 and HLA-DR is reduced, in average, to at most 80%, 65%, 70% and 80%, respectively, of the positive control. The same assay can be used for other MSCs.

In one embodiment, the ASCs or other MSCs suppress the proliferation of PBMCs, e.g., as determined in a Mixed Lymphocyte Reaction (MLR). This type of assay is well-known in the art, and may comprise mixing ASCs or other MSCs with stimulated PBMCs from an allogeneic donor in different ratios, *e.g.,* in the range 1:20 to 1:1, using PBMCs without ASCs as positive controls, and measuring after a 4-day co-culture period, the PBMC incorporation of 3H-thymidine (25 μSi/ml) during an 18-20 h incubation period. Using this type of assay, as compared to the positive control, a 1:20, 1:10, 1:5 and 1:1 ratio of ASCs to PBMCs may result in an average 3H-thymidine incorporation of at most about 80%, 75%, 55%, and 25%, respectively, of the positive control.

In some embodiments, the ASCs or other MSCs are also or alternatively characterized by specific markers indicative of immunomodulation, especially immunosuppression, such as CD10, CD140a, CD160, CD204, CD258, CD270, CD272, CD44, CD49a, CD54, CD9, Galectin 3, Galectin 9, HLA-G, LTβR and combinations thereof. Without being limited to theory, these markers are associated with immune signalling, cell-cell and cell-ECM adhesion, homing, pattern recognition, T cell inhibition, up-regulation of growth factor receptors and inactivation of pro-inflammatory proteins.

In a further embodiment, the ASCs or other MSCs are also or alternatively characterized by a change in one or more cell surface markers in response to a pro-inflammatory cytokine such as interferon-gamma. For example, it has been shown that immunosuppression by human-derived MSCs is mediated by indoleamine 2,3-dioxygenase (IDO) and that human MSCs express high levels of IDO in response to inflammatory cytokines (Ren et al., Stem Cells 2009 Aug;27(8): 1954-62). Accordingly, in some embodiments, the expression of IDO reflects the immunosuppressive ability of the ASC or other MSC population to inhibit T -cells proliferation. The immunomodulatory profile or potency can, for example, be determined by the assay described in Example 1, incubating the ASC or other MSC population in the presence of IFN-gamma (e.g., 25 ng/mL IFN-gamma for 24 hours) and then determining the IDOl expression (e.g., using an anti-IDOl antibody) in comparison with a control. The control may be, for example, the ASC or other MSC population incubated without IFN-gamma, the IDO expression of the ASC or other MSC population before incubation with IFN-gamma, a known control value, or the like.

In another embodiment, upon interferon-gamma stimulation according to Example 11 of WO 2017/068140 A1, the percentage of the ASC population expressing CD274 is increased to at least 80% and the expression level of CD54 on CD54-positive cells is increased at least 25-fold.

In some embodiments, the composition is from a thawed, ready-to-use preparation of cryopreserved allogeneic adult human ASCs free of non-human animal proteins. In a preferred embodiment, the composition comprises a suspension of allogeneic adult human ASCs in a protein-free cryoprotectant at a concentration of at least about $1.5 \times 10^7$ allogeneic adult human ASCs per mL. In some embodiments, the concentration is from about $2 \times 10^7$ to about $5 \times 10^7$ allogeneic adult human ASCs per mL in a protein-free cryoprotectant. For example, in one embodiment, the concentration is from about $1.8 \times 10^7$ to about $2.6 \times 10^7$, such as from about $2.0 \times 10^7$ to about $2.4 \times 10^7$, such as about $2.2 \times 10^7$ ASCs per mL in a protein-free cryoprotectant. In one embodiment, the concentration is from about $4 \times 10^7$ to about $5 \times 10^7$, such as from about $45 \times 10^6$ to about $5 \times 10^7$, such as about $50 \times 10^6$ ASCs per mL in a protein-free cryoprotectant.

In some embodiments, the ready-to-use preparation has a total volume of up to 5 mL, such as from about 0.5 to about 2 mL, such as from about 1.0 mL to about 1.5 mL, such as about 1.1 mL or 1.3 mL. In one specific embodiment, the ready-to-use preparation has a total volume of about 1.3 mL and comprises about 29 million allogeneic adult human ASCs suspended in added protein-free cryoprotectant.

The cryoprotectant used for preparing the compositions is typically protein-free, endotoxin-free and sterile.

In the clinical study described in Example 2, a cryopreserved ASC product (CSCC_ASC2210) based on high-concentration human allogeneic ASCs and a protein-free cryoprotectant comprising 10% dimethyl sulfoxide (DMSO) can be safe and effective when administered to a burn wound. So, in one embodiment, the cryoprotectant comprises DMSO, preferably at a concentration of about 5% to about 15% (v/v). In one embodiment, the cryoprotectant comprises about 5%, about 6%, about 8%, about 10%, about 12% or about 15% DMSO. Preferably, the cryoprotectant comprises about 5% DMSO (v/v) or about 10% DMSO (v/v).

Alternatively, the DMSO can be replaced by a glucan such as, for examples dextran, having an average molecular weight in the range of 35000 to 45000 Da, such as, e.g., Dextran-40.

In one embodiment, the cryoprotectant comprises a 1:10 to about 1:20 mixture of DMSO and an aqueous solution comprising

> (a) one or more electrolytes selected from the group consisting of potassium ions at a concentration ranging from about 35-45 mM, sodium ions ranging from about 80-120 mM, magnesium ions ranging from about 2-10 mM, and calcium ions ranging from about 0.01-0.1 mM;
> (b) a macromolecular oncotic agent having a size sufficiently large to limit escape from the circulation system and effective to maintain oncotic pressure equivalent to that of blood plasma and selected from the group consisting of human serum albumin, polysaccharide and colloidal starch;
> (c) a biological pH buffer effective under physiological and hypothermic conditions;
> (d) a nutritive effective amount of at least one simple sugar;
> (e) an impermeant and hydroxyl radical scavenging effective amount of mannitol;
> (f) an impermeant anion impermeable to cell membranes and effective to counteract cell swelling during cold exposure, said impermeant ion being at least one member selected from the group consisting of lactobionate, gluconate, citrate and glycerophosphate;
> (g) a substrate effective for the regeneration of ATP, said substrate being at least one member selected from the group consisting of adenosine, fructose, ribose and adenine; and
> (h) glutathione.

In one embodiment, the cryoprotectant comprises a 1:10 to about 1:20 mixture of DMSO and an aqueous solution comprising

> a) one or more electrolytes selected from the group consisting of potassium ions at a concentration ranging from 35-45 mM, sodium ions ranging from 80-120 mM, magnesium ions ranging from 2-10 mM, and calcium ions ranging from 0.01-0.1 mM;
> b) a macromolecular oncotic agent having a size sufficiently large to limit escape from the circulation system and effective to maintain oncotic pressure equivalent to that of blood plasma and selected from the group consisting of human serum albumin, polysaccharide and colloidal starch;
> c) a biological pH buffer effective under physiological and hypothermic conditions;
> d) a nutritive effective amount of at least one simple sugar;
> e) an impermeant and hydroxyl radical scavenging effective amount of mannitol;
> f) an impermeant anion impermeable to cell membranes and effective to counteract cell swelling during cold exposure, said impermeant ion being at least one member selected from the group consisting of lactobionate, gluconate, citrate and glycerophosphate;
> g) a substrate effective for the regeneration of ATP, said substrate being at least one member selected from the group consisting of adenosine, fructose, ribose and adenine, and
> h) at least one agent which regulates apoptotic induced cell death.

While several suitable cryoprotectants are commercially available or otherwise known in the art, non-limiting examples of cryoprotectants contemplated for the ASC compositions of the present invention are CryoStor® (BioLife Solutions), including CryoStor CS2, CryoStor CS5 and CryoStor CS10; and ProFreeze (Lonza). CryoStor freeze media are sterile serum-free and protein-free, having a pH 7.5 - 7.7, and an endotoxin level under 1 EU/mL. In one embodiment, the cryoprotectant is Hypothermosol® (CMS, Rockville, Md.) plus 10% DMSO (WO 2000/002572 A1). Hypothermosol® comprises Trolox (6-hydroxy-2,5,7,8-tetramethylchroman-2-carboxylic acid), $Na^+$, $K^+$, $Ca^{2+}$, $Mg^{2+1}Cl^-$, $H_2PO_4^-$, HEPES, lactobionate, sucrose, mannitol, glucose, Dextran-40 (*i.e.,* dextran with an average MW of 40,000 Da), adenosine and glutathione (WO 2010/064054 A1). According to the manufacturer, ProFreeze should be supplemented with 10% DMSO at time of use. WO 2000/002572 A1 and WO 2010/064054 A1 are hereby incorporated by reference in their entireties.

In one specific embodiment, the cryoprotectant in which the ASCs are suspended is protein-free and comprises DMSO at a concentration of about 5% to about 15% (v/v), such as about 5% (v/v) or 10% (v/v), and Trolox (6-hydroxy-2,5,7,8-tetramethylchroman-2-carboxylic acid), $Na^+$, $K^+$, $Ca^{2+}$, $Mg^{2+}$, $Cl^-$, $H_2PO_4^-$, HEPES, lactobionate, sucrose, man-

nitol, glucose, dextran-40, adenosine and glutathione.

In one specific embodiment, the composition comprises a suspension of allogeneic adult human ASCs at a concentration of about $2.2 \times 10^7$ per mL, the composition prepared by suspending allogeneic adult human ASCs in a protein-free cryoprotectant comprising about 10% (v/v) DMSO and, optionally, Trolox (6-hydroxy-2,5,7,8-tetramethylchroman-2-carboxylic acid), $Na^+$, $K^+$, $Ca^{2+}$, $Mg^{2+}$, $Cl^-$, $H_2PO_4^-$, HEPES, lactobionate, sucrose, mannitol, glucose, dextran-40, adenosine and glutathione.

In one specific embodiment, the composition comprises a suspension of allogeneic adult human ASCs at a concentration of about $2.2 \times 10^7$ per mL, the composition prepared by suspending allogeneic adult human ASCs in a protein-free cryoprotectant comprising about 5% (v/v) DMSO and, optionally, Trolox (6-hydroxy-2,5,7,8-tetramethylchroman-2-carboxylic acid), $Na^+$, $K^+$, $Ca^{2+}$, $Mg^{2+}$, $Cl^-$, $H_2PO_4^-$, HEPES, lactobionate, sucrose, mannitol, glucose, dextran-40, adenosine and glutathione.

Alternatively, in any one of the preceding embodiments, the cryoprotectant is DMSO in a v/v concentration between about 1% to about 15%, such as about 5% or about 10%, in a chemically-defined serum-free and xeno-free media suitable for ASCs, such as, e.g., Eagles Basal Medium or Dulbecco's Modified Eagle's Medium (DMEM). Such cryoprotectants are described in WO 2017/144552 A1.

In one preferred embodiment, the compositions for use as described herein are obtained or obtainable by a process comprising the steps of

(i) adding the stromal vascular fraction (SVF) of a lipoaspirate collected from a human donor to a bioreactor wherein at least one surface is pre-treated to promote adhesion of adult human stem cells;
(ii) in the bioreactor, cultivating adherent cells to confluence in a serum-free culture medium supplemented with human platelet lysate;
(iii) detaching the adherent cells;
(iv) freezing the detached cells in a cryoprotectant at a concentration of at least $1 \times 10^6$ cells/mL;
(v) thawing the frozen cells and repeating steps (ii) and (iii) at least once,
(vi) freezing the detached cells in a cryoprotectant at a concentration of at least $1.5 \times 10^7$ cells/mL; and
(vii) thawing the frozen composition.

In one embodiment, the lipoaspirate in step (i) is obtained from abdominal adipose tissue from the donor.

In one embodiment, in step (ii), at least one surface of the bioreactor protein is pre-treated with a composition comprising or consisting of cryoprecipitate. Cryoprecipitate is a well-known blood product prepared from plasma, *e.g.,* where fresh plasma is frozen and thawed and the precipitate collected. The product typically contains fibrinogen and Factor VIII, as well as *e.g.* von Willebrand factor, Factor XIII and fibronectin. In some embodiments, the cryoprecipitate contains at least 140 mg or more of fibrinogen per 70 IU of Factor VIII, optionally prepared from either AB or low-titer A blood donors.

In one embodiment, in step (ii), the serum-free culture medium comprises about 5% human platelet lysate. In a specific embodiment, the serum-free culture medium is a minimal essential medium (*e.g.,* Minimum Essential Medium, MEM Alpha (aMEM) without Ribonucleosides and Deoxyribonucleosides, (Gibco, Life Technologies)) supplemented with 1% Penicillin/Streptomycin (*e.g.,* Gibco, Life Technologies) and about 5% human platelet lysate (*e.g.,* Stemulate, Cook General Biotechnology).

In step (v), if steps (ii) and (iii) are repeated more than once, step (iv) may be conducted in between each round, *i.e.,* so that there is a freezing step after each detaching step.

In one specific embodiment, the composition is prepared as described in Example 1 of WO 2017/068140 A1, except that the total volume of the final CSCC_ASC may be less than 5 mL, such as between 0.25 mL and 1.5 mL, and/or the concentration of the ASCs may be higher, such as about $2 \times 10^7$ per mL or higher, e.g., up to about $5 \times 10^7$ per mL.

In one specific embodiment, the composition comprises about $1 \times 10^7$ ASCs per mL, about $2 \times 10^7$ ASCs per mL or about $5 \times 10^7$ ASCs per mL suspended in a protein-free cryoprotectant comprising about 5% DMSO, e.g., in CryoStor® CS5 (BioLife Solutions). In a preferred embodiment, the composition is prepared as described in Example 1.

Also provided are the compositions obtained when thawing the frozen ASC compositions. The frozen ASC compositions may, for example, be thawed in a 37°C water bath or thawed/stored in room temperature in the operation room.

In some embodiments, when determined immediately after thawing of the composition, at least about 80%, such as at least 85%, such as at least 90%, such as at least 95%, such as at least 98% of the ASCs are viable, as determined by dye exclusion methods known in the art. For example, the DNA-binding fluorescent dye propidium iodide can be added to the cells, and an image cytometer used to determine the proportion of non-fluorescent cells. Preferably, at least 90% of the cells are viable.

As for proliferation capacity, in some embodiments, when placed in culture immediately after thawing of the composition, the ASCs can be characterized by a population doubling (PD) of at least 1, such as at least 1.3, such as at least 1.5, such as at least 1.7, such as at least 2, when cultured in tissue culture flasks for 48h (*e.g.,* according to the method in Example 3 of WO 2017/068140 A1). Preferably, the ASCs have a PD of at least 1, such as at least 1.5. PD is calculated as Ln (N)/Ln 2, where N = Cell harvested/Cell seeded.

Preferred are ASC compositions where, immediately after thawing

(a) at least 85% of the ASC population are viable cells, and the viability after storage in room temperature for 2 hours is at least 80%;
(b) the ASC population has a proliferation capacity providing for a PD of at least 1 when cultured for 48 hours;
(c) the ASC population is capable of suppressing dendritic cell maturation and activation;
(d) the recovery after thawing is over 95%, and the recovery of cells after storage at room temperature for 2 h after thawing is at least 85%
(e) the ASC population has an *in vitro* cell adherence such that at least 60%, such as at least 65%, such as at least 70% of the total number of cells are adherent after 5h in cultivation.

However, also additional and/or alternative components are contemplated for the compositions. Specifically, the pharmaceutical composition may further comprise a soluble biomaterial or hydrogel containing natural or synthetic biopolymers such as extracellular matrix proteins, -peptides, -glycosaminoglycans or other compounds including, but not limited to, collagen, alginate, agarose, hyaluronic acid derivatives, chitosan, and fibrin. In one embodiment, the pharmaceutical composition may comprise sterile and endotoxin free Alginate (Sodium alginate VLVG, Novamatrix, FMC Biopolymers, Norway), partially calcium cross-linked with D-gluconic acid and hemicalcium salt (Follin et al., 2015). For example, the alginate can be mixed with an ASC or other MSC preparation and a cryoprotectant to a final concentration of 1 % (w/v) partially cross-linked alginate before the final cryopreservation step. In another embodiment, partially cross-linked alginate is stored at RT and mixed with the final product to a final concentration of 1% (w/v) alginate, e.g., by injecting the ASC preparation into the alginate container. In another embodiment, the pharmaceutical composition may comprise an ASC or other MSC preparation mixed with fibrin, e.g., in the form of fibrin glue (see, e.g., Mehanna et al., Biomed Res Int. 2015; 2015: 846062) or a fibrin polymer spray system (Falanga et al., Tissue Eng. 2007; 13, 1299-1312). Such compositions may be particularly suitable for topical administration of stem cells to a burn wound or other skin lesion.

Therapeutic applications

The compositions disclosed herein are useful for treating subjects, particularly human subjects, with a wound or other skin lesion in need of treatment. The compositions are also useful for prophylactic treatment, i.e., where a subject is at risk for developing a skin lesion, such as, e.g., a scar resulting from a wound or a wound resulting from prolonged pressure on the skin. Other, non-limiting examples of skin lesions include ulcers and rashes. Particularly contemplated for treatment according to the aspects and embodiments described herein are thermal burn wounds.

A subject, typically a patient, may present with one skin lesion or several skin lesions of the same or different origin. In cases where a subject has several skin lesions, the skilled practitioner will be able to determine which one or more skin lesions are suitable for treatment according to the invention at a particular point in time, e.g., depending on the nature, severity and cause of each skin lesion as well as the condition of the subject. Skin lesions to be treated according to any aspect or embodiment herein may be of any shape or size, e.g., having an area of at least 1 cm$^2$, such as at least 2 cm$^2$, such as at least 4 cm$^2$, such as at least 10 cm$^2$, such as at least 20 cm$^2$, such as at least 30 cm$^2$, such as at least 40 cm$^2$ or more. In some embodiments, the skin lesion is at least 60 cm$^2$, at least 80 cm$^2$, at least 120 cm$^2$ or more.

In some embodiments, the skin lesion is caused by an injury, such as a physical/mechanical, thermal, electrical, chemical or radiation injury. A physical/mechanical injury may, for example, lead to an abrasion, avulsion, contusion, crush wound, cut, laceration, projectile wound or a puncture wound. Thermal, electrical, chemical or radiation injury may lead to a burn wound, typically characterized according to the injury as a thermal burn, electrical burn, chemical burn, or radiation burn. In some embodiments, the skin lesion for treatment according to the invention is a thermal burn wound or a radiation burn wound.

In some embodiments, the skin lesion is caused by a disease or disorder, e.g., a disease or disorder selected from an inflammatory disease or disorder, an autoimmune disease or disorder, an allergic or hypersensitivity disease or disorder, a degenerative disease or disorder, an ischemic disease or disorder, a rheumatic disease or disorder, a metabolic disease or disorder, an endocrine disease or disorder or a benign neoplastic disease or disorder. In some embodiments, the skin lesion for treatment according to the aspects and embodiments disclosed herein is caused by a skin disease or disorder selected from alopecia (baldness and hair loss), including, but not limited to male- and female-pattern hair loss, alopecia areata and telogen effluvium; epidermolysis bullosa, dermatitis, scleroderma, lupus, psoriasis, rosacea, atopic dermatitis, lichen sclerosus, lichen planus and lymphedema. For treatment of, e.g., dermatitis, see e.g.

Sah et al., Allergy. 2018 Dec;73(12):2364-2376, who reported that subcutaneous injection of MSCs improved symptoms in patients with moderate to severe atopic dermatitis (AD).

In some embodiments, the skin lesion is a wound, i.e., a break in the continuity of the skin with loss of the epidermis and at least part of the dermis. Without being limited to theory, MSCs have been shown to enhance wound healing through increased angiogenesis, re-epithelialization, and granulation tissue formation (e.g., Isakson et al., 2015). A wound to be treated according to any aspect or embodiment herein may be of any shape or size, e.g., having an area of at least 1 cm$^2$, such as at least 2 cm$^2$, such as at least 4 cm$^2$, such as at least 10 cm$^2$, such as at least 20 cm$^2$, such as at least 30 cm$^2$, such as at least 40 cm$^2$ or more. In some embodiments, the wound has an area of at least 60 cm$^2$, at least 80 cm$^2$, at least 120 cm$^2$ or more.

In some embodiments, the skin lesion is an ulcer or chronic wound. Generally, this may occur when there is a failure of injured skin to proceed to heal through an orderly and timely process to produce anatomic and functional integrity. Chronic wounds may include, for example, diabetic skin ulcers, pressure sores, surgical wounds, burn wounds, and ischemic wounds.

Particularly contemplated for treatment according to the invention are burn wounds, including, but not limited to, thermal burn wounds.

The depth of a burn wound is prognostic of whether it can heal conservatively or will require surgery. As set out more fully in Table 3, burns are therefore classified depending on the lesion depth into four categories: (1) superficial, also termed epidermal or first degree with painful skin redness; (2) superficial partial-thickness, also termed superficial-dermal or second degree burns, associated with painful blisters and an underlying pink and wet or weeping wound surface that blanch on pressure; (3) deep partial-thickness, also termed deep-dermal or second degree burns, also associated with painful blisters but with an underlying fairly dry wound surface which is usually mottled pink and white in colour, and less or no blanching on pressure; and (4) full-thickness, also termed subdermal or third degree burns, which are usually brown, black or waxy and white in appearance with a dry leathery and firm texture and are anaesthetic because of nerve damage, in some cases presenting with visible clotted vessels in the depth. Superficial/first-degree burns affect only the epidermis, and resolve without intervention in a few days without scarring. Superficial partial-thickness burns involve the epidermis and the superficial part of the dermis (papillary dermis), and usually heal within 14 days, typically without scarring. Deep partial-thickness burns involve the epidermis and the deep layer of the dermis (reticular dermis), with the basal membrane completely destroyed. Healing typically takes more than 21 days and usually results in scarring. Full thickness burns involve all the layers of the skin and may include damage to subdermal structures such as fat, muscle, cartilage and bone. Healing is slow and typically takes more than 21 days, usually with considerable scarring. Because the skin appendages, such as hair follicles, sebaceous and sweat glands are eventually destroyed in both deep partial-thickness and full-thickness burns, these deep burns will not heal spontaneously by epithelialisation from these structures within the wound, but only from the periphery i.e. wound edge. This may be associated with prolonged healing time and significant scarring. Scar contractures, i.e., a tightening of the scarred skin, may occur in both deep partial-thickness and full-thickness burns and may be functionally debilitating, e.g. restricting motion if situated close to joints. For wounds that are not anticipated to heal within 3 weeks, or are at risk for not healing within 3 weeks, conventional treatment usually rely on surgery with excision of the eschar/necrotic tissue (i.e., escharectomy or necrectomy) and subsequent autologous skin grafts for quick restoration of the skin barrier function.

Unlike mechanical injuries, burns are dynamic. Primary tissue loss in a burn injury is seen nearest the source of insult and arises from rapid cell death because of cellular protein denaturation and coagulation, following thermal, chemical, electrical, friction, or radiation-induced burns. A burn injury develops and is categorized into three zones of decreasing injury severity: 1) the zone of coagulation, 2) the zone of stasis and 3) the zone of hyperaemia. The central zone of coagulative necrosis results from immediate cellular denaturation and cell death by the burn insult. Surrounding the zone of coagulation is the indirectly affected and initially viable zone of stasis where the damage in the tissue is less severe, and consequently, immediate cell death does not occur. However, the zone of stasis is characterized by complex interrelated pathophysiological processes, such as excessive and harmful inflammatory response; oxidative stress; and decreased tissue perfusion and ischemia. The outermost zone of hyperaemia, which surrounds the zone of stasis, comprises non-injured tissue that is characterized by vasodilation, increased blood flow and hyperaemia as a compensatory reaction to the burn injury. Tissue in the zone of hyperaemia invariably recovers. Tissue in the zone of stasis is potentially salvageable, given timely and proper intervention. If not timely and properly treated, however, the tissue in the zone of stasis dies as a result of e.g. necrosis and/or apoptosis, as the above-mentioned pathophysiological processes further compromises blood flow to already critically injured/ischemic tissues. In this scenario, the zone of stasis coalesces with and extends the zone of coagulation. Clinically, this is seen as progression of the depth of the burn injury, a phenomenon in the art know as burn wound progression, where areas of burns that initially appear viable, subsequently (3-5 days post-burn) become necrotic.

Prior to treatment of a burn wound according to the aspects and embodiments describe herein, standard initial burn management may be performed, such as one or more of the administration of pain medication, cooling of burns with tap water, removal and deroofing of loose skin and blisters, respectively, wound cleaning with water and neutral soap, and wound dressing and bandaging (see Example 2). In some embodiments, the compositions as described herein are used for treating a burn wound not subjected to prior escharectomy or necrectomy.

Preferably, treatment, i.e., administration of the composition, is initiated within no more than about 48 hours of the burn injury, such as no more than within about 42 hours of the burn injury, such as no more than within about 40 hours of the burn injury, such as no more than within about 36 hours of the burn injury, such as no more than within about 34 hours of the burn injury, such as no more than within about 32 hours of the burn injury, such as no more than within about 30 hours of the burn injury, such as no more than within about 28 hours of the burn injury, such as no more than within about 24 hours of the burn injury, such as no more than within about 20 hours of the burn injury, such as no more than within about 12 hours of the burn injury. In a preferred embodiment, the treatment is initiated no more than about 36 hours from the burn injury.

The composition can be administered to the burn wound by any suitable route. Preferably, however, the composition is provided to the burn wound by local administration, e.g., by intradermal injection, subcutaneous injection, or topical application. Preferably, the composition is administered by intradermal injection, as described in more detail elsewhere herein.

Different types of burn wounds are contemplated for the present treatment methods. The type of burn wound and its suitability for treatment according to the aspects and embodiments described herein can be determined by the skilled practitioner by clinical examination, which is usually a specialized physician, such as a burn expert. The skilled practitioner may also or alternatively utilize routine techniques known in the art for the determination, e.g., routine Laser Doppler Imaging (LDI)-scan. The LDI device generates a color-coded perfusion map that can classify burns into three healing potentials: High, < 14 days; Intermediate, 14-21 days; and low, >21 days. As determined by the skilled practitioner upon the time of presentation, a burn wound to be treated according to the methods described herein is typically a partial-thickness burn. The partial-thickness burn may, for example, be determined to be a deep partial-thickness burn, a superficial partial-thickness burn, or a superficial-to-deep partial thickness burn. In some embodiments, the burn wound is determined to be a deep partial-thickness burn wound. In some embodiments, the burn wound is a partial-to-full-thickness burn wound, or a full-thickness burn wound.

As described above, burn wounds are at risk for progression. So, the aspects and embodiments described herein can be used for preventing the progression of a burn wound, such as a partial-thickness burn wound. Accordingly, in some embodiments, the burn wound is determined to be a partial-thickness burn wound at risk for progression. For example, in some embodiments, the burn wound may be determined to be a partial-thickness burn wound at risk for progression into a full-thickness burn wound. In some embodiments, the burn wound is determined to be a deep partial-thickness burn wound at risk for progression into a full-thickness burn wound. In some embodiments, the burn wound is determined to be a superficial partial-thickness burn wound at risk for progression into a deep partial-thickness burn wound and/or a full-thickness burn wound. In some embodiments, the burn wound is determined to be a superficial-to-deep partial-thickness burn wound at risk for progression into a deep partial-thickness burn wound and/or a full-thickness burn wound.

In some embodiments, treatment of burn wounds according to the aspects and embodiments disclosed herein results in a reduced extent of tissue damage or necrosis of the burn wound.

The extent of tissue damage or necrosis can be quantified by well-known methods, e.g., by measuring the quantitative degree of necrosis in a skin-punch biopsy of the burn wound as described in Example 2, using microscopic evaluation or immunohistochemical staining with a HMGB1 antibody. The skin-punch biopsy can be taken pre-treatment and at one or more suitable time points post-treatment, e.g., at 14 days. In particular embodiments, the extent of tissue damage or necrosis of the burn wound is decreased by at least 5%, such as at least 10%, such as at least 15%, such as at least 20%, such as at least 25%, such as at least 30%, such as at least 40%, such as at least 50%, compared to a control. The control may be, for example, a burn wound, or an area of the same burn wound, that is not treated with a stem cell composition, or a reference or standard value.

In some embodiments, treatment of burn wounds according to the aspects and embodiments disclosed herein results in a reduced extent of apoptosis in the burn wound. The extent of apoptosis can be quantified by well-known methods, e.g., by measuring the caspase activation in a skin-punch biopsy of the burn wound as described in Example 2, using microscopic evaluation and immunohistochemical staining. In particular embodiments, the extent of apoptosis of the burn wound is decreased by at least 5%, such as at least 10%, such as at least 15%, such as at least 20%, such as at least 25%, such as at least 30%, such as at least 40%, such as at least 50%, compared to a control. The control may be, for example, a burn wound or an area of the same burn wound that is not treated with a stem cell composition, or a reference or standard value.

In some embodiments, treatment of burn wounds according to the aspects and embodiments disclosed herein results in a reduced extent of local inflammation in the burn wound. The extent of local inflammation can be quantified by well-known methods, e.g., by measuring the quantitative degree of inflammatory cell infiltration with inflammatory cells such as neutrophils, monocytes and macrophages in a skin-punch biopsy of the burn wound as described in Example 2, using microscopic evaluation or immunohistochemical staining, such as with myeloperoxidase. The skin-punch biopsy can be taken pre-treatment and at one or more suitable time points post-treatment, e.g., at 14 days. In particular embodiments, the extent of local inflammation in the burn wound is decreased by at least 5%, such as at least 10%, such as at least 15%, such as at least 20%, such as at least 25%, such as at least 30%, such as at least 40%, such as at least 50% compared to a control. The control may be, for example, a burn wound or an area of the same burn wound that is not treated with a stem cell composition, or a reference or standard value.

In some embodiments, treatment of burn wounds according to the aspects and embodiments disclosed herein results in an improved immunomodulation in the burn wound. The improvement in immunomodulation can be quantified by well-known methods, e.g., by quantitatively measuring the subject's cellular response of cytokines such as downregulation of pro-inflammatory and/or upregulation of anti-inflammatory cytokines and growth hormones in a skin-punch biopsy of the burn wound, e.g. as described in Example 2, using immunoassays and qPCR. The skin-punch biopsy can be taken pre-treatment and at one or more suitable time points post-treatment, e.g., at 14 days. In particular embodiments, the extent of pro-inflammatory cytokines and anti-inflammatory cytokines (and growth hormones) in the burn wound is decreased and increased, respectively, by at least 5%, such as at least 10%, such as at least 15%, such as at least 20%, such as at least 25%, such as at least 30%, such as at least 40%, such as at least 50%, compared to a control. The control may be, for example, a burn wound or an area of the same burn wound that is not treated with a stem cell composition, or a reference or standard value.

In some embodiments, treatment of burn wounds according to the aspects and embodiments disclosed herein results in an increased revascularisation and/or angiogenesis of the burn wound. The extent of revascularization can be quantified by well-known methods, e.g., by microscopic evaluation or immunohistochemical staining with a CD31 antibody of a skin-punch biopsy of the burn wound as described in Example 2. The skin-punch biopsy can be taken pre-treatment and at one or more suitable time points post-treatment, e.g., at 14 days. In particular embodiments, the extent of revascularization of the burn wound is increased by at least 5%, such as at least 10%, such as at least 15%, such as at least 20%, such as at least 25%, such as at least 30%, such as at least 40%, such as at least 50%, compared to a control. The control may be, for example, a burn wound or an area of the same burn wound that is not treated with a stem cell composition, or a reference or standard value.

In some embodiments, treatment of burn wounds according to the aspects and embodiments disclosed herein results in an increased blood perfusion and as such healing potential of the burn wound. The extent of blood perfusion can be quantified by well-known methods, e.g., by Laser Doppler Imaging (LDI) of the burn wound as described in Example 2. The LDI measurement can be made pre-treatment and at one or more suitable time points post-treatment, e.g., at 30, 60, 90 or 120 minutes or 5, 10 or 14 days. In particular embodiments, the extent of blood perfusion of the burn wound is increased by at least 5%, such as at least 10%, such as at least 15%, such as at least 20%, such as at least 25%, such as at least 30%, such as at least 40%, such as at least 50%, compared to a control. The control may be, for example, a burn wound or an area of the same burn wound that is not treated with a stem cell composition, or a reference or standard value.

In some embodiments, treatment of burn wounds according to the aspects and embodiments disclosed herein results in an increased and/or accelerated regeneration of skin appendages and normal anatomical structures of the skin of the burn wound. This can be quantified by well-known methods, e.g., by microscopic evaluation of a skin-punch biopsy of the burn wound as described in Example 2. The skin-punch biopsy can be taken pre-treatment and at one or more suitable time points post-treatment, e.g., at 14 days. In particular embodiments, the regeneration of normal structural skin anatomy of the burn wound is increased and/or accelerated by at least 5%, such as at least 10%, such as at least 15%, such as at least 20%, such as at least 25%, such as at least 30%, such as at least 40%, such as at least 50% compared to a control. The control may be, for example, a burn wound or an area of the same burn wound that is not treated with a stem cell composition, or a reference or standard value.

In some embodiments, treatment of burn wounds according to the aspects and embodiments disclosed herein results in an increased degree and/or rate of healing of the burn wound. The degree and/or rate of healing can be quantified by well-known methods, e.g., by measuring the reduction in wound surface area over time as described in Example 2. The wound surface area can be measured pre-treatment and at one or more suitable time points post-treatment, e.g., at 5, 10 and/or 14 days. In particular embodiments, the degree and/or rate of healing of the burn wound is increased by at least 5%, such as at least 10%, such as at least 15%, such as at least 20%, such as at least 25%, such as at least 30%, such as at least 40%, such as at least 50%, compared to a control. The control may be, for example, a burn wound or an area of the same burn wound that is not treated with a stem cell composition, or a reference or standard value.

In some embodiments, treatment of burn wounds according to the aspects and embodiments disclosed herein results in reduced need for later skin grafting, as determined by the skilled practitioner. In particular embodiments, the rate of later skin grafting is reduced by at least 5%, such as at least 10%, such as at least 15%, such as at least 20%, such as at least 25%, such as at least 30%, such as at least 40%, such as at least 50%, compared to a control. The control may be, for example, a burn wound or an area of the same burn wound that is not treated with a stem cell composition, or a reference or standard value.

In some embodiments, treatment of burn wounds according to the aspects and embodiments disclosed herein results in a reduced extent of scarring and/or fibrosis of the burn wound. This can be quantified by well-known methods, e.g., by microscopic evaluation of a skin-punch biopsy of the burn wound as described in Example 2. The skin-punch biopsy can be taken pre-treatment and at one or more suitable time points post-treatment, e.g., at 14 days. In particular embodiments, the extent of scarring and/or fibrosis is reduced by at least 5%, such as at least 10%, such as at least 15%, such as at least 20%, such as at least 25%, such as at least 30%, such as at least 40%, such as at least 50% compared to a control. The control may be, for example, a burn wound or an area of the same burn wound that is not treated with a stem cell composition, or a reference or standard value.

In some embodiments, treatment of burn wounds according to the aspects and embodiments disclosed herein results in an increased quality of the scar from the burn wound as compared to a control, as determined by the skilled practitioner. The skilled practitioner may utilize routine techniques known in the art for the determination, e.g., POSAS scale (see www.posas.org) or a skin analysis device, measuring traditional skin quality parameters, such as e.g. elasticity, hydration, pigmentation and transepidermal waterloss (TEWL). The control may be, for example, a burn wound or an area of the same burn wound that is not treated with a stem cell composition, or a reference or standard value.

Particularly contemplated is a composition comprising allogeneic ASCs for use according to any aspect or embodiment herein, in treating a burn wound in the skin of in a human subject, comprising administration of from about $1 \times 10^4$ to about $1 \times 10^6$ ASCs per cm$^2$ of a burn wound, without prior escharectomy or necrectomy.

Administration

The skin, which is the body's largest organ by surface area, is easily accessible from the surface by injectable drug delivery in the treatment of various skin lesions, including wounds, ulcers, scars and rashes. The stem cell compositions for the therapeutic applications disclosed herein are administered in a manner compatible with their formulation, and in a therapeutically effective amount. The quantity to be administered depends, for instance, on the skin lesion, skin disease or skin disorder to be treated or prevented, the health status of the subject, the objective of the stem cell therapy, etc. The precise amount of therapeutic composition to be administered to a particular skin lesion is determined by the skilled practitioner and can be adapted to each individual subject or patient. However, at least in some embodiments, suitable dosages of the stem cell compositions may range from about from about $1 \times 10^4$ to about $1 \times 10^6$ stem cells per cm$^2$ of skin lesion or skin area per treatment occasion, particularly when the stem cells are ASCs or other MSCs. Contemplated are also regimens where an initial administration is followed by one or more repeated doses at suitable intervals, e.g., on a weekly or monthly basis.

In some embodiments, the stem composition is administered topically, e.g., onto a burn wound. The compositions may, for example, be administered by applying the stem cell compositions to a scaffold (e.g., including but not limited to a biocompatible synthetic or non-synthetic matrix, such as a skin substitute), and applying the scaffold seeded with the stem cell composition to the burn. Alternatively, in some embodiments, a scaffold (e.g., including but not limited to a biocompatible synthetic or non-synthetic matrix, such as a skin substitute) is applied to the burn, and the stem cell compositions are applied onto the scaffold. Other methods of administering the stem cell compositions as disclosed herein include, but are not limited to, those described in Gerlach, et al. Burns 2011;37:e19-e23. In this method, the regenerative cells are placed into a sterile syringe with a fitted nozzle, and sprayed directly through the nozzle onto the burn wound. Using computer-assisted delivery, the gun distributes the cells at a uniform velocity throughout the wound. Such a method could also readily be used to apply the stem cell compositions to a scaffold as described herein.

In some embodiments, the stem cell composition is administered by intradermal or subcutaneous injection using one or more injection needles into the skin lesion or skin area at risk for a skin lesion. The compositions may, for example, be injected intradermally or subcutaneously into a skin lesion or skin area at risk for a skin lesion every 0.01 cm$^2$, 0.02 cm$^2$, 0.03 cm$^2$, 0.04 cm$^2$, 0.05 cm$^2$, 0.06 cm$^2$, 0.07 cm$^2$, 0.05 cm$^2$, 0.06 cm$^2$, 0.07 cm$^2$, 0.08 cm$^2$, 0.09 cm$^2$, 0.10 cm$^2$, 0.11 cm$^2$, 0.12 cm$^2$, 0.13 cm$^2$, 0.14 cm$^2$, 0.15 cm$^2$, 0.16 cm$^2$, 0.17 cm$^2$, 0.18 cm$^2$, 0.19 cm$^2$, 0.2 cm$^2$, 0.25 cm$^2$, 0.3 cm$^2$, 0.4 cm$^2$, 0.5 cm$^2$, 0.6 cm$^2$, 0.7 cm$^2$, 0.8 cm$^2$, 0.9 cm$^2$, 1 cm$^2$, 2 cm$^2$, 3 cm$^2$, or 4 cm$^2$. In some embodiments, the composition may, for example, be administered at 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 needle insertion sites per cm$^2$ skin. In some embodiments, the composition may, for example, be administered at between 2 to 27 needle insertion sites per cm$^2$ of skin, such as between 3 and 18 insertion sites per cm$^2$ of skin, such as between 5 and 15 insertion sites per cm$^2$ of skin, such as between 7 and 11 insertion sites per cm$^2$ of skin. In particular embodiments, the compositions are administered at 5 or 9 needle insertion sites per cm$^2$.

The dosage of cells to be administered at each needle insertion site is typically determined by the physician or other skilled practitioner responsible for the treatment, typically so as to deliver a therapeutically effective dosage per cm$^2$ or other area unit of the skin lesion or skin area to be treated. In some embodiments, the composition according to any aspect or embodiment is administered at a dosage per injection needle insertion site of at least about $1 \times 10^3$ stem cells, at most about $1 \times 10^6$ stem cells or both, i.e., a dosage per injection needle in the range of about $1 \times 10^3$ to about

$1 \times 10^6$ stem cells. In some embodiments, the composition is administered at a dosage of at least about $1 \times 10^3$ stem cells and at most about $2 \times 10^3$, about $4 \times 10^3$, about $6 \times 10^3$, about $8 \times 10^3$, about $1 \times 10^4$, about $2 \times 10^4$, about $4 \times 10^4$, about $6 \times 10^4$, about $8 \times 10^4$, about $1 \times 10^4$, about $1 \times 10^5$, about $2 \times 10^5$, about $4 \times 10^5$, about $6 \times 10^5$, about $8 \times 10^5$ or about $1 \times 10^6$ stem cells per needle insertion site. In particular embodiments, the composition is administered at a dosage per injection needle insertion site of at least about $1 \times 10^3$ stem cells and at most about $2 \times 10^5$ stem cells, such as at most about $1 \times 10^5$ stem cells.

The area to be treated with the stem cell compositions described herein is typically determined by the physician or other skilled practitioner responsible for the treatment, based on previous diagnostic evaluation of the skin lesion or skin area to be subjected to prophylactic treatment. In embodiments where a skin lesion is to be treated, the physician may, for example, determine that the composition is only to be administered to part of the skin lesion, to the entire skin lesion, or to the entire skin lesion as well as to skin areas adjacent to the skin lesion. For example, in the case of a partial-thickness burn wound, e.g., a partial-thickness burn wound without a visible zone of coagulation, the responsible physician may determine that the composition is to be administered to the entire burn wound area, including the zone of stasis and the zone of hyperemia; to the zone of stasis and the zone of hyperemia and adjacent areas of the skin; or only to the zone of stasis.

In some embodiments, conventional intradermal or subcutaneous injection using a syringe and hypodermic needle is used for the administration. Conventional intradermal injection usually involves substantially parallel needle insertion to the skin at a five to fifteen-degree angle to the skin. Subcutaneous injection can also be achieved by conventional techniques well-known to the skilled practitioner. The skilled artisan will readily appreciate that various devices, e.g., the JUVAPEN™ injection device (Juvaplus, SA, Switzerland), the intradermal injection systems provided by Terumo Co. (Tokyo, Japan; e.g., Immucise®) and the like, as well as injection devices similar to that described by Leoni et al. (2017) can also be used for this purpose.

As used herein, in the context of an angle between an injection needle inserted into the skin and the skin surface, the angle is the smaller angle between a plane defined by the skin at the site of the injection needle insertion point and the axis running along the length of a shaft of the needle.

Alternatively, however, stem cell compositions can be delivered to dermis, which is located right under the epidermis, or to the subcutaneous tissue, which is located right under the dermis, by a shorter needle. The needle is then placed at a higher angle, typically at an angle of at least about 60 degrees to the skin surface, which allows the dermis to be reached by use of an intradermal injection device. Advantages of such drug delivery systems may include less pain experienced by the subject, ease of operating the device, and a safer injection technique due to the shorter injection route in the dermis. In addition, automated injection devices may have some advantages over manual injection techniques, regarding better control over volumetric dosing and faster delivery. Before implementation of novel injectable cell-based therapeutics in the clinical, qualifying a suitable delivery protocol is critical to ensure post-ejectional cellular health and uniform cell distribution, to provide reproducible and optimal therapeutic efficacy.

As described herein and illustrated in Example 1, the present inventors have now identified parameters applicable to automated injection of stem cells, particularly ASCs and other MSCs, allowing for reproducible injections of stem cells while preserving acceptable health and potency. The experiments reported in Example 1 were carried out with the handheld, automatic dermal injection gun Vital Injector 2 from EunSung Global Corporation (Seoul, South Korea), using a sterile 5- or 9-multi needle head with needles of 31G, showing safe and accurate treatments defined by e.g., total injection volume, depth of needle penetration, speed of injection and needle retraction latency in the skin after injection. The Vital Injector 2 is described at www.eunsung.ro/products/vital-injector/. Briefly, however, the device is composed of two main units: the console and the interconnected handheld injection gun. To shorten the time of treatment, the device makes use of a disposable vacuum-assisted 9- or 5- multi-needle array head of 31G, which is mounted onto the device and connected to an interchangeable syringe of choice (1, 2, 3 or 5 cc). The following parameters can be adjusted on the console's touch screen by the operator: 1) injection mode (intermittent or continuous), 2) dose (intermittent : 5 - 30 µL or continuous: 1 cc: 1, 2, 3 cc/min; 2, 3 or 5 cc: 1, 3, 5 cc/min), 3) needle depth (0.0 - 5.0 mm, intervals: 0.2 mm), 4) speed/time needles stays inserted in the skin (1 - 6 sec, to account for fluid viscosity and thus allow the time required for viscous fluids to reach the tip of needles) and 5) vacuum level (arbitrary: 1 - 5, from weakest to strongest, to the keep skin taut during needle insertion). However, as the skilled artisan will realize, other delivery devices suitable for automated injections can be readily adapted and used for this purpose based on the present disclosure and the results in Example 1.

More specifically, the delivery device is preferably a delivery device comprising plurality of substantially parallel injection needles, wherein the device is adapted to deliver a liquid formulation, optionally a viscous liquid formulation, through the plurality of substantially parallel injection needles when inserted into the skin. Typically, the delivery device is capable of or adapted to intradermal or subcutaneous injection of mesotherapy agents, fillers, hyaluronic acid/skin boosters and the like. Particularly, the delivery device is capable of delivering a liquid formulation comprising a suspension of stem cells at a concentration of between about $1 \times 10^6$ per mL to about $1 \times 10^8$ per mL. More particularly, the delivery device is capable of delivering a liquid formulation comprising a suspension of stem cells at a concentration of between

about $1 \times 10^7$ to about $5 \times 10^7$ per mL, such as from about $1 \times 10^7$ to about $4 \times 10^7$ per mL; preferably wherein the stem cells are MSCs, such as ASCs.

Moreover, the delivery device is preferably adapted to keep the skin area in and around the insertion site of each injection needle taut during at least the needle insertion and injection phases, optionally by creating a vacuum, suction or mechanical force to gently lift the skin towards a surface comprising an opening from which the injection needle emerges. This may be accomplished by a vacuum chamber, e.g., that the device comprises a vacuum chamber comprising at least the tip of each injection needle. A vacuum chamber may for example, be placed in the needle head of the delivery device, creating a tensile and/or compressive force on the skin to keep it taut.

Using such a delivery device, in a particular embodiment, the composition can be administered by a method comprising the steps of:

(a) applying the device to a predetermined area of the skin, wherein the device is loaded with a volume of the composition and activated to create a vacuum, suction or mechanical force to gently lift the skin towards a plurality of surfaces, each surface comprising an opening from which an injection needle can emerge;

(b) activating the device to

i. insert the tip of each injection needle into a predetermined injection needle insertion/injection depth in the skin; and

ii. inject from each injection needle a predetermined volume of the composition;

(c) optionally, repeating steps (a) and (b) at an adjacent or other predetermined area of the skin.

In some embodiments, step (b)(ii) or both of steps (b)(i) and (ii) can be repeated at least once, such as twice or three times, before proceeding to step (c), so as to inject a larger volume of the stem cell composition.

Typically, step (c) is then repeated until the composition has been administered to the desired area of the skin lesion or skin area.

Preferably, the one or more injection needles are inserted into the skin at an angle of at least about 60 degrees to the skin surface, such as at least about 70 degrees to the skin surface, such as at least about 75 degrees to the skin surface, such as at least about 80 degrees to the skin surface, such as at least about 85 degrees to the skin surface, such as substantially perpendicular to the skin surface. For example, the one or more injection needles may be inserted into the skin at an angle of at least about 80, such as at least about 81, such as at least about 82, such as at least about 83, such as at least about 84, such as at least about 85, such as at least from about 86, such as at least about 87, such as at least about 88, such as at least about 89, such as at about 90 degrees to the skin surface. In some embodiments, the one or more injection needles are inserted into the skin at an angle which is substantially perpendicular to the skin surface, e.g., at an angle of at least about 80 degrees to the skin surface.

The injection parameters are adjusted so as to achieve intradermal and/or subcutaneous delivery of the desired dosage of stem cells or stem cell composition while maintaining stem cell health and potency. Non-limiting injection parameters include the following:

*Injection needle insertion depth.* As used herein, the "needle insertion depth" is the length of needle inserted into skin, i.e., the length of needle from the needle tip to the point of entry of the needle in the skin, whereas the "needle injection depth" refers to the shortest vertical distance from the needle tip (or opening) to the skin surface. The needle insertion depth and the needle injection depth are approximately equal when the injection angle is 90 degrees to the skin surface, differing only in the distance from the needle opening to the needle tip. The needle insertion/injection depth parameter is adjusted so as to achieve, e.g., optimal skin piercing and dermal delivery and minimal degree of reflux for the specific stem cell composition to be administered, the injection angle to be used, and the combined thickness of the epidermis and dermis in the skin lesion or skin area to be treated. For example, in case intradermal delivery is desired, a needle insertion/injection depth should be chosen which leaves the needle tip (or opening) in the dermis. Conversely, in case subcutaneous delivery is desired, a needle insertion/injection depth should be chosen so that the needle opening is located in subcutaneous tissue. So, in various embodiments, the needle insertion/injection depth can be, for example, about 0.2 mm, 0.4 mm, 0.6 mm, 0.8 mm, 1.0 mm, 1.2 mm, 1.4 mm, 1.6 mm, 1.8 mm, 2.0 mm, 2.2 mm, 2.4 mm, 2.6 mm, 2.8 mm, 3.0 mm, 3.2 mm, 3.4 mm, 3.6 mm, 3.8 mm, 4.0 mm, 4.2 mm, 4.4 mm, 4.6 mm, 4.8 mm or 5.0 mm. In some embodiments, the injection needle insertion depth is at least about 0.6 mm, such as at least about 1 mm, such as at least about 1.5 mm, such as at least about 1.6 mm. Preferably, when the composition comprises a suspension of ASCs or other MSCs at a concentration of about $1 \times 10^7$ to about $5 \times 10^7$ cells/mL, such as about $2.2 \times 10^7$ cells/mL in a cryopreservative such as 5% or 10% DMSO, optionally in the form of CryoStor CS5 or CryoStor CS10, the injection needle insertion/injection depth is at least 1.0 mm, such as at least 1.6 mm. In some embodiments, the injection angle is substantially perpendicular to the skin.

*Injection volume.* The injection volume per needle, and thereby injection volume per needle insertion site, is chosen so that the desired dosage of stem cells is delivered from a stem cell composition of a specific concentration of stem cells. The injection volume may, for example, be at least about 0.5 microliter ($\mu$L), at most about 50 $\mu$L, or both, i.e., in the range of about 0.5 $\mu$L to at most about 50 $\mu$L. In some embodiments, the injection volume may, for example, be at least about 0.5 microliter ($\mu$L), at most about 30 $\mu$L, or both, i.e., in the range of about 0.5 $\mu$L to at most about 30 $\mu$L. In some embodiments, the injection volume may, for example, be at least about 0.5 microliter ($\mu$L), at most about 20 $\mu$L, or both, i.e., in the range of about 0.5 $\mu$L to at most about 20 $\mu$L. In some embodiments, the injection volume is about 1, 2, 3, 4, 5, 6, 7, 8, 9, 10, 11, 12, 13, 14, 15, 16, 17, 18, 19 or 20 $\mu$L. In some embodiments, the composition is administered at a volume per injection needle insertion site of from about 1 $\mu$L to about 10 $\mu$L, such as from about 2 $\mu$L to about 5 $\mu$L. In some embodiments, particularly when the composition comprises a suspension of ASCs or other MSCs at a concentration of about $1 \times 10^7$ to about $5 \times 10^7$ cells/mL, such as about $2.2 \times 10^7$ cells/mL in a cryopreservative such as 5% or 10% DMSO, optionally in the form of CryoStor CS5 or CryoStor CS10, the injection volume per needle may be about 3.3 $\mu$L or about 6 $\mu$L. In some embodiments, particularly when the composition comprises a suspension of ASCs or other MSCs at a concentration of about $1 \times 10^7$ to about $5 \times 10^7$ cells/mL, such as about $2.2 \times 10^7$ cells/mL in a cryopreservative such as 5% or 10% DMSO, optionally in the form of CryoStor CS5 or CryoStor CS10, the injection volume per needle may be about 3.3 $\mu$L or about 5 $\mu$L.

*Injection rate.* The composition is injected at a rate that maintains acceptable cellular health and potency. Preferably, the composition is administered at an injection rate per needle of no more than about 1.0 mL/min, such as no more than about 0.5 mL/min, such as no more than about 0.4 mL/min, such as no more than about 0.3 mL/min, such as no more than about 0.25 mL/min, such as no more than about 0.22 mL/min, such as no more than about 0.2 mL/min, such as no more than about 0.15 mL/min, such as no more than about 0.1 mL/min. In some embodiments, particularly when the composition comprises a suspension of ASCs or other MSCs at a concentration of about $1 \times 10^7$ to about $5 \times 10^7$ cells/mL, such as about $2.2 \times 10^7$ cells/mL in a cryopreservative such as 5% or 10% DMSO, optionally in the form of CryoStor CS5 or CryoStor CS10, the injection rate may be no more than about 0.4 mL/min, such as no more than about 0.22 mL/min.

*Needle retraction latency in skin.* Since stem cell compositions according to the aspects and embodiments described herein may have a high concentration or density of suspended cells, resulting in a certain viscosity or density. To allow for some delay in the actual ejection of the stem cell compositions from each needle, the needle can advantageously remain inserted for a period of time after the immediate injection phase. For example, after the injection or injection phase, each needle can remain inserted into the skin for a period of at least 2 seconds, such as at least 3 seconds, such as at least 4 seconds, such as at least 5 seconds, such as at least 6 seconds, such as at least 7 seconds, such as at least 10 seconds, before retraction. Preferably, the needle remains inserted into the skin for at least about 5 seconds, such as at least 6 seconds, before retraction.

*Needle dimensions.* The needle dimensions may affect the shear to which the stem cells are exposed during the injection phase, and may thereby also affect the cell health and potency post-injection, although the shear stress may also depend on, e.g., the concentration of stem cells in the composition. Moreover, the needle dimensions, particularly the needle diameter, should be chosen so that needle clogging is minimized, particularly for highly concentrated stem cell compositions. Preferably, the composition is administered via one or more injection needles having an inner diameter of at least about 0.1 mm, at most about 0.5 mm, or both, i.e., an inner diameter in the range of about 0.1 mm to about 0.5 mm. For example, the composition is administered via one or more injection needles having an inner diameter of more than about 0.1 mm, optionally having a Gauge (G) selected from 25G, 26G, 27G, 28G, 29G, 30G, 31G, 32G and 33G. In some embodiments, an injection needle having at least the inner diameter of a 31G needle is used for a composition comprising a suspension of ASCs or other MSCs at a concentration of about $1 \times 10^7$ to about $5 \times 10^7$ cells/mL, such as about $2.2 \times 10^7$ cells/mL in a cryopreservative such as 5% or 10% DMSO, optionally in the form of CryoStor CS5 or CryoStor CS10. Further, in some embodiments, the length of the injection needle through which the stem cell composition must pass is at most about 3 cm, such as a length of at most 2 cm, such as a length of at most about 1.8 cm.

Administration by intradermal injection is particularly contemplated for the stem cell compositions for therapeutic methods according to the aspects and embodiments described herein, particularly for a composition comprising a suspension of ASCs or other MSCs at a concentration of about $1 \times 10^7$ to about $5 \times 10^7$ cells/mL, such as about $2.2 \times 10^7$ cells/mL in a cryopreservative such as 5% or 10% DMSO, optionally in the form of CryoStor CS5 or CryoStor CS10.

The post-ejection cell health and potency can be studied according to methods known in the art or according to methods described in Example 1. The latter include (1) Perceived cell viability (count and recovery), determining the percentage of cells that do not have a ruptured cell membrane; (2) Long-time cell viability, determining the percentage of cells that have not died during a predetermined period, e.g., in the range of 12h-24h; (3) Adherence/spreading, determining the percentage of cells with high eccentricity value after plating and incubation in complete media; (4) Proliferation, determining confluency, lag-phase and the population doubling time (PDT), (5) Metabolism; and (6) Immunomodulatory profile, determining the difference in IDOI expression in live cells; typically using as the control the same stem cell composition not subjected to ejection.

The delivery efficacy of a stem cell composition can be evaluated according to methods known in the art or according to methods described in Example 1. The latter include (1) Needle clogging assay, determining the needle clogging tendency by whether the number of ejection droplets of stem cell composition from a plurality of injection needles decrease after repeated ejections; (2) Detecting needle penetration, intradermal delivery and/or fluid reflux along the penetration tract when injecting e.g. stem cell composition or methylene blue (MB) solution into human abdominal skin; (3) Histological examination of stem cell composition injected into human abdominal skin.

The following Examples are illustrative only, and should not be interpreted as limiting.

EXAMPLE 1

The rationale for this study was to assess intradermal MSC-delivery efficiency, using an injectable drug delivery device. For this purpose, an automated dermal injection gun (Vital Injector2, EunSung Global Corporation, Seoul, South Korea) was used. The injection gun has been commercially developed for mesotherapy to inject low to high viscous formulations (e.g. skin boosters) in skin rejuvenation (anti-age treatment). We hypothesized that such a multi-use injectable device could be used in MSC therapy as a novel cell delivery device, and therefore conducted the present validation study.

The objectives were to evaluate the cell delivery device with respect to:

1) Cellular health and potency post-ejection of various concentrations of MSC-suspensions in (*in vitro* experiment).

2) Functionality: needle clogging, tissue penetration and intradermal delivery efficiency and reflux after injection, *i.e.,* factors potentially affecting the distribution efficiency in human skin and thereby the predictability of the treatment results (*ex vivo* experiment).

*1. Materials and methods*

1.1. Drug delivery device and stem cell preparation

The injectable drug delivery device; Vital Injector2 (VI2) is composed of a console with an integrated touch screen and an interconnected handheld injection gun, onto which a syringe is loaded.

To shorten the time of treatment, the device makes use of a disposable vacuum-assisted 9- or 5- multi-needle array head of 31G, which is mounted onto the injection gun and connected to an interchangeable syringe of choice (1, 2, 3 or 5 cc). The following parameters can be adjusted on the console's touch screen by the operator: 1) injection mode (intermittent or continuous), 2) dose (intermittent: $5 \sim 30$ µl or continuous: 1cc: 1, 2, 3 cc/min; 2, 3 or 5 cc: 1, 3, 5 cc/min), 3) needle depth ($0.0 \sim 5.0$ mm, intervals: 0.2 mm), 4) speed/time needles stays inserted in the skin ($1 \sim 6$ sec, to account for fluid viscosity and thus allow the time required for viscous fluids to reach the tip of needles) and 5) vacuum level (arbitrary: $1 \sim 5$, from weakest to strongest, to the keep skin taut during needle insertion).

Schematic representations of the 9- and 5-multi-needle arrays are shown in **Fig. 16A** and **Fig. 16B,** respectively.

Stem cell preparation:

Human adipose-derived mesenchymal stromal/stem cells (ASCs) from four donors were expanded in complete media (DMEM (22320-030, Gibco) with 10% pooled human platelet lysate, 2 IE/ml heparin (741827, Amgros I/S), and 1% penicillin-streptomycin (15140-122, Gibco)) until reaching approximately 80% confluence. At day of harvest, cells were washed with PBS w/o Mg/Ca and detached using trypsin (TrypLe, Gibco). The enzyme was inactivated with complete media and the cells pelleted. Following resuspension in complete media, cells were counted using Vial cassettes on a NC3000 (Chemometec). The cells were pelleted once more and resuspended in CryoStor® CS5 (BioLife Solutions) in vials of 10-20-50 x$10^6$ ASCs. The vials were cryopreserved using a controlled-rate freezer (Kryo 560-16; Planer PLC, cooling program: start temp. 4°C, -1°C/min drop to 0°C, -2°C/min drop to -45°C, and - 5°C/min drop to -100°C) and stored in liquid nitrogen until use. The cell viability precryopreservation was >90%.

1.2. Assessment of post-ejection cell health and potency

Cells were thawed by submerging the vials into 37°C water bath for 2 min. When completely thawed, cells were gently homogenized and aspirated into a 1 ml syringe and immediately ejected (2 cc/min) with the VI2 through 5 or 9 pin 31G cannulas (each n=4). Following ejection, the cells were collected for further analysis. Baseline was defined as cell viability of post thawed samples prior to ejection through cannulas. Viability of all baseline samples were >85%.

1.2.1 Perceived cell viability (count and recovery)

Ejection of cells in higher concentration may lead to mechanical damage and ruptured cell membrane, which is easily detected with acridine orange and DAPI nuclear staining. Cell count and viability were performed with Via-1 cassettes on an NC-3000 (Chemometec). Recovery was defined as the fraction of total cell count in the ejected samples when compared with the corresponding baseline sample.

1.2.2 Long-time cell viability (progression of cell death)

Cells with an intact membrane immediately post-thaw and post-ejection may die from freeze- and shear stress-related cell damage over time. The cytotoxicity assay detects accumulated cell death over 12 hours. Thawed, ejected cells were dyed with Cytotox red (Essen Biosciences), seeded in 96 well cell culture (167008, Nunc) and placed in an Incucyte (Essence Bioscience) for 12 hours for hourly monitoring of cell death. Using the Incucyte Software, accumulated cell death was estimated as the fraction of dead cells of total seeded cells.

1.2.3 Adherence/spreading

Shear stress may prolong the time a cell takes to recover when introduced into to a new environment. Cell adhesion/spreading was defined as a cell losing the initial rounded shape (low eccentricity value). Thawed ejected cells were seeded in 96 cell culture plates (167008, Nunc) in complete media and incubated in the Incucyte (Essence Bioscience). The fractions of cells with high eccentricity value (cut-off value 0.8) were calculated using the Incucyte software.

1.2.4 Proliferation (vitality/potency)

Stressed cells often have a prolonged lag phase in culture and sometimes even an impaired population doubling time (PDT). Proliferation can be used as a measure of both vitality and potency. Thawed ejected cells were seeded in replica wells in 96 well cell culture plates (167008, Nunc) in complete media. The plate was placed in an Incucyte (Essence Bioscience) to monitor the cell confluency on an hourly basis. Confluency is defined as the amount of space occupied by cells. The proliferation curves were analyzed using the Shiny app (RStudio), and the PDT was calculated using the following formula:

$$T(h) = (\log(2))/(\log(1+(\text{growth rate}/100)). \quad (I)$$

1.2.5 Metabolism (vitality)

Stressed cells often have reduced metabolism. Thawed ejected cells were seeded in replica wells in 96 well cell culture plates (167008, Nunc) in complete media added Presto Blue (Sigma) and placed in the cell incubator. Following 4 hours, changes in OD 570nm were determined.

1.2.6 Immunomodulatory profile (potency)

When ASCs become stressed their ability to induce IDO expression might diminish and as such their immunosuppressive ability to inhibit T-cells proliferation. Briefly, to investigate this, thawed ejected ASCs were cultured in complete media supplemented with 25 ng/mL IFN-gamma and cultured for 24 hours. The cells then harvested and stained with 7AAD (live/dead marker) and anti-IDOI (AF647-conjugated) antibody and changes in IDOI expression was subsequently analyzed by flow cytometry (Navios, Beckman Coulter) and Kaluza 2.1 analysis-software (Beckman Coulter).

1.3. Assessment of distribution efficiency in human skin for predictable treatment results

To functionally validate the VI2, the needle clog tendency of successive ejections was assessed, using cell suspensions of 10-20-50 x10$^6$ASCs/ml.

Furthermore, discarded human skin from abdominoplasty ("tummy tuck") was used within 1 hour after surgery for injections to assess:

- Efficiency of needle penetration and intradermal delivery; degree of reflux along the penetration tract; and visualization using high frequency ultrasound of methylene blue dye in the dermis.
- Deposition of thawed cells in the skin.

1.3.1. Clogging assay

To evaluate needle clogging tendency of cell suspension of various cell concentration and thereof viscosity, three cell concentrations (thawed cryopreserved 10, 20 and 50 $\times 10^6$ ASCs/ml in CryoStor® CS5) were ejected using either the 9- or the 5 pin multi-needle head. Fifteen successive 30 µl ejections were made onto a fluid-repellent surgical draper and the number of pin droplets for each administration was quantified macroscopically. A missing pin droplet was interpreted to result from a clogged pin.

1.3.2. Experimental mounting of human skin to ensure in vivo skin elasticity

Discarded human abdominal skin including approximately 2-4 cm of subcutaneous tissue, was circumferentially secured onto cork plates using 25G cannulas. To ensure consistent stretch of the skin during tissue mounting, an elasticity probe (DermaLab Combo, Cortex Technology, Hadsund, Denmark) was systematically used to model *in vivo* skin elasticity measurements.

1.3.3.1 Experimental setup using methylene blue injections in human skin

For the purpose of detecting needle penetration of the skin, intradermal delivery and fluid reflux along the penetration tract, methylene blue 2% (MB) solution was used as a staining dye. Staining of the dermis occurs because hydrophilic MB dye binds to proteins in the tissue. Once the hydrophobic stratum corneum barrier of the skin is breached, the MB dye diffuses through the dermis and provides bright stained tissue (Moronkeji et al., J. Control. Release 2018;265:102-112). Because of the hydrophobic nature of lipids, fat cells are not readily stained by MB, although, the vessels and fibers of connective tissue of adipose tissue are stained (Genina et al., In: Proc. SPIE 2004;5486). In the present study, MB solution was aspirated into a 1 ml syringe, loaded onto the VI2 with the syringe connected to either a 9- or a 5- pin multi-needle head. The following settings on the VI2 device were used to inject into skin: intermittent injection mode; dose: 30 µl; speed: 6 sec; vacuum level: 5. Successive skin injections were made at six pre-specified injection depths of 0.6, 0.8, 1.0, 1.6, 2.0 and 2.6 mm. To account for and visualize any pre- or post-injectional needle obstruction (i.e. clogging tendency) in between skin injections, a deposition of MB was made onto a fluid-repellent surgical draper, before and after each skin injection. In case no needle obstruction occurred, 9 (or 5) equally-sized droplets were deposited onto the hydrophobic surface following each administration.

1.3.3.2 Experimental setup using ASC-injections stained with methylene blue in human skin

The same experimental setup and settings on the VI2 were used for ASC-injections, as described above in section 1.3.3.1, except that the first two pre-specified injection depths of 0.6, 0.8 were deliberately omitted to only include 1.0, 1.6, 2.0 and 2.6 mm. Following thawing, a 20 $\times 10^6$ ASCs/ml suspension was stained with MB and gently aspirated into a 1 ml syringe, before intradermally injected with the VI2, using a 9 and pin head.

1.3.4. Measurement of fluid reflux

To evaluate the degree of fluid reflux as a function of injection depth, refluxed dye at the surface of the skin was absorbed using a small white filter paper by gently placing it on top of the skin with forceps. At the end of the experiment, filter papers where placed on a white background alongside a ruler, photographed with a high-resolution digital camera in a standardized fashion, and uploaded to a computer-assisted image analysis software (ImageJ). The surface area of the absorbed dye was calculated in $mm^2$ by computerized planimetry.

1.3.5. Needle penetration efficiency

Following completion of the injection series (n=10) and collection of fluid reflux, the skin surface was gently wiped clean with an ethanol pad. A videomicroscope (DermaLab Combo, Cortex Technology, Hadsund, Denmark) was used to scan the surface of the skin for MB staining (seen as blue coloured dots) to verify successful transepidermal needle breach of the stratum corneum. The penetration efficiency (PE) was calculated as follows:

$$PE\ (9\ pin) = \frac{n\ blue\ dots}{9}\ \text{x}\ 100\%\ \text{(II)}$$

$$PE\ (5\ pin) = \frac{n\ blue\ dots}{5}\ \text{x}\ 100\%\ \text{(III)}$$

### 1.3.6. Skin Ultrasound

A high frequency (20 MHz transducer) skin ultrasound (DermaScan C USB, Cortex Technology, Hadsund, Denmark) was used to give real-time and high-resolution imaging of the dermally injected depositions (at ~5 min post-injection). Snapshots were taken for documentation.

### 1.3.7. Efficiency of intradermal delivery

To evaluate the efficiency of intradermal delivery, the dermis was macroscopically examined for MB staining following skin dissection. The skin was vertically incised down to the subcutaneous tissue with a surgical scalpel, intersecting each of the three arrays of needle penetration sites per administration for both 9- and 5-pin multi-needles. The three resulting tissue blocks were excised and oriented with their right dermal surface facing upwards for quantification of dermal tissue stains per administration.

The deposition efficiency (DE) was calculated as follows:

$$DE\ (9\ pin) = \frac{n\ intradermal\ blue\ stains}{9}\ \text{x}\ 100\%\ \text{(IV)}$$

$$DE\ (5\ pin) = \frac{n\ intradermal\ blue\ stains}{5}\ \text{x}\ 100\%\ \text{(V)}$$

Briefly, excised tissue blocks from discarded human skin tissue were used to macroscopically evaluate the efficiency of intradermal delivery of the injectable drug delivery device. After a series of gradually deeper intradermal injections of methylene blue through either a 9 or 5 pin multi-needle head, the skin was vertically incised down to the subcutaneous tissue, intersecting the needle penetration sites of each array. Three resulting tissue blocks per administration were excised, each consisting of the full thickness of the skin including the superficial layer of the subcutaneous tissue. Tissue blocks were oriented with their right dermal surface facing upwards for quantification of number of dermal tissue stains (blue) per administration.

### 1.3.8. Histology

Skin tissue was subsequently injected with ASCs to confirm successful intradermal cell delivery histologically.
Following thawing, $20 \times 10^6$ ASCs were stained with methylene blue 2% and gently aspirated into a 1 ml syringe. The syringe was loaded onto the VI2 and 30 $\mu$l of the cell suspension injected into human skin (n=1) using both 9- and 5-pin heads. The following settings on the VI2 were used: intermittent mode; dose: 30 $\mu$l; needle depth: 2.0 mm; speed: 6 sec; and vacuum level: 5.
Tissue specimens (~ 1 x 1 x 1.5 cm) with the injection sites, consisting of skin and subcutaneous tissue, were excised, sliced, formalin fixed, paraffin embedded, cut, mounted on glass slides and stained with haematoxylin and eosin (H&E). Histological examination in light microscope was performed by an experienced pathologist.

### 1.4. Statistical analysis

All statistical analysis were performed using GraphPad Prism (GraphPad Software Inc, La Jolla, CA). A p-value < 0.05 was considered statistically significant.

*2. Results*

2.1. Post-ejection cell health and potency

The cellular health and potency of three ASC concentrations - 10, 20 and 50 x$10^6$ ASCs/ml - were evaluated in vitro, following dispensation using either a 5 or 9 pin head, as previously described. Baseline samples refers to cells analyzed post thaw without being ejected through cannulas and serves in addition, also as quality control of freezing/thawing procedures.

All samples where cryopreserved with viability >90% and the post-thaw perceived viability of >85% (baseline). Overall a tendency towards lower post-thaw viability was observed in the highest cell concentrations.

No significant difference (p>0.05) were observed in post-ejectional perceived viability, recovery and metabolism vs baseline of each cell concentration, using either a 9- or 5-pin head (**Fig. 1**). However, the long-time cell viability (accumulated cell death over 12 hours) declined significantly regardless of cell concentration when ejected through both the 9 pin (p=0.0043) and 5 pin (p=0.0002) head when compared with baseline (**Fig. 2** and **Table 1**).

Table 1

| Dunnett's multiple comparisons test for the results shown in **Fig. 2**: | | | | | |
|---|---|---|---|---|---|
| | Mean Diff. | 95,00% CI of diff. | Significant? | Summary | Adjusted P-value |
| Baseline vs. 5 pin | -10.66 | -16.00 to -5.313 | Yes | *** | 0.0002 |
| Baseline vs. 9 pin | -7.808 | -13.15 to -2.463 | Yes | ** | 0.0043 |

Although no significant difference was observed in adherence and hours in lag phase when culturing the dispensed cells, a tendency to prolonged time to reach confluence were observed in cells ejected through the 5 pin head (**Fig. 3**). This observation was confirmed when assessing the population doubling time, as a significant prolonged PDt was seen when cells were ejected through 9 pin or 5 pin compared to baseline (p<0.0001 or p=0.0063, respectively), with 9 pin being the least evasive (**Fig. 4** and **Table 2**).

Table 2

| Dunnett's multiple comparisons test for the results shown in **Fig. 4**: | | | | | |
|---|---|---|---|---|---|
| | Mean Diff. | 95,00% CI of diff. | Significant? | Summary | Adjusted P-value |
| Baseline vs. 5 pin | -11.10 | -15.24 to -6.965 | Yes | **** | <0.0001 |
| Baseline vs. 9 pin | -5.767 | -9.902 to -1.631 | Yes | ** | 0.0063 |

Administration through the drug delivery device did not affect the investigated ASCs immunomodulatory potency as there was no significant difference in IDOI expression, regardless of either cell concentration or type of multi-needle head used (**Fig. 5**).

2.2. Distribution efficiency in human skin

Once acceptable post-ejectional cell health and potency had been confirmed *in vitro,* we next sought to evaluate the drug delivery device for predictable delivery, penetration and deposition in human skin.

2.2.1. Needle clogging

To evaluate the clogging tendency, ASC suspensions of 10, 20 and 50 x$10^6$ cells/ml were consecutively ejected through either the 9-pin or the 5-pin and the number of droplets counted. While the 10 and 20 x$10^6$ cells/ml suspensions demonstrated no clog tendency, the suspension of 50 x$10^6$ cells/ml showed a propensity to clog the needles of both 9 and 5 pins when the same multi-needle head was used for repeated ejections (first clog at the 4th and 8th successive ejection, respectively) (**Fig. 6**).

2.2.2. Piercing, dermal delivery and degree of reflux as a function of depth

To validate successful cutaneous delivery of gradually deeper injections, MB solution was used as trace dye to assess skin penetration, dermal deposition and reflux in human skin, as previously described. Using an injection depth above 1.0 mm (1.6-2.6 mm) resulted in all needles piercing the skin (PE 100%), with dermal deposition from 7-8 needles with minimal reflux to the skin surface (**Fig. 7**). Good results were also seen with the 5 pin, though already from a depth of 1 mm. (**Fig. 8**). Similar trends were observed in a preliminary study using ASC-injections and either a 9- or 5-pin needle head (**Fig. 9**). However, while coloration of target tissue was also preliminarily evaluated, the results were not deemed optimally quantitative since the staining of the cell suspension was too weak for adequate sensitivity in when macroscopically examined.

2.2.3. Skin ultrasound: Site-specific delivery

The dermally injected MB fluid and ASC depositions were visualized and confirmed by high frequency skin ultrasound imaging. Imaging revealed delineated black spaces within the dermis following injections, interpreted as sites of successful intradermal delivery containing pools of fluid (Gupta et al., Biomaterials 2011;32:6823-6831). Furthermore, echographs of gradually deeper injections showed a trend of moving deeper in the dermis.

2.2.4. Histology

Histological evaluation confirmed the presence of injected ASCs within the dermis of excised skin tissue. Histology showed sites of closely packed cells within the target site, i.e., dermis with normal surrounding tissue architecture, representing the injected ASCs.

*3. Conclusion*

In our study no significant difference was observed in post-ejectional perceived viability (i.e. ruptured cell membranes) vs baseline of even the highest cell concentration of 50 x$10^6$ ASCs per ml, using either multi-needle head.
The higher cell concentration seemed to be less viable when ejected through cannulas.
Mean long-time cell viability appeared lower for 5- vs. 9 pin, supported by longer time to reach confluency (e100%; estimated confluency), although not significant.
A significant prolonged population doubling time was also observed when cells were ejected through 9 pin or 5 pin, with 9 pin being the least evasive.
Overall, the long-term viability results were all within the defined range of acceptance (post-thaw viability >70%). See Gramlich et al., Sci. Rep. 6, (2016); Baust et al., In Vitro Cellular and Developmental Biology - Animal 53, 855-871 (2017); Guidance for FDA Reviewers and Sponsors: Content and Review of Chemistry, Manufacturing, and Control (CMC) Information for Human Gene Therapy Investigational New Drug Applications (INDs). in (U.S. Dept. of Health and Human Services, Food and Drug Administration, Center for Biologics Evaluation and Research, 2008); and Davies et al., Cryobiology 69, 342-347 (2014).
Successively deeper intradermal injections gave less reflux along the penetration tract created by needle insertion compared to more superficial injections, as confirmed in the skin experiment, and was accompanied with an increased intradermal delivery efficiency. The efficiency of intradermal deposition of the 9 and 5 pin needle system was ≥ 90% at a minimum depth of 1.6 mm and 1.0 mm, respectively.

EXAMPLE 2

The objective of this study is to test, in a triple-blinded, randomized, placebo-controlled trial, if early local application of culture-expanded allogeneic ASCs in acute burns of *deep* partial-thickness can limit the extent of the local inflammation and damage/necrosis, promote spontaneous and accelerated healing of the skin, reducing the need of conventional skin grafting (ultimately, if deep partial-thickness burns can entirely avoid being skin grafted) and/or improve the quality of the scar.
Specifically, the application of allogeneic ASCs can have an anti-inflammatory, anti-apoptotic and regenerative effect on deep partial-thickness burns, and can therefore:

> 1) Limit the extent of local inflammation, as well as the progressive damage and necrosis of the burns, by early intervention in the acute phase (≤ 36 hours post-burn)
> 2) Promote spontaneous healing, and consequently:

>> a. Accelerate healing rate with earlier wound closure
>> b. Reduce the area in need of coverage with split-skin grafts
>> c. Regenerate the skin and its appendages

3) Improve the quality of wound healing; preventing unsightly scarring and improve traditional skin quality parameters. If scars appear, the quality of these can be improved and of better quality compared to the scars following deep burns we see today.

*Study design*

The design is a triple-blinded, randomized placebo-controlled paired study, on competent adult burn patients, with each serving as their own control (eliminating any inter-individual differences).

Initially, a pilot study, with the same study design and endpoints as the main study, is conducted to test feasibility, safety and logistics, before committing to the main study.

Inclusion Criteria

- Age ≥18 years, of either gender
- Hospitalized patients with clinically deep partial-thickness acute thermal burns, presenting as two separate areas (each at least 6 x 6 cm), or an area large enough to fit two 6 x 6 cm areas, located symmetrically on the same or opposite anatomical region on the truncus or extremities
- The intervention must be possible to be initiated within 36 hours of the acquired burn injury

The deep partial-thickness burns are divided into a clinically homogenous pair of 6 x 6 cm areas and randomly treated (according to a computer-generated concealed allocation sequence) by injecting one of the following:

1. Advances therapy investigational medicinal product (ATIMP): Suspension of allogeneic ASCs (previously culture-expanded and cryopreserved)
2. Placebo (composition, apart from ASCs is otherwise identical to the ATIMP): excipient CryoStor® CS10).

Description of Study Drug

The study drug (CSCC_ASC2210), is an ATIMP holding allogeneic adipose-tissue derived mesenchymal stromal cells from healthy donors.

The study drug is manufactured according to GMP at Cardiology Stem Cell Centre, Rigshospitalet, using manual isolation of cells from abdominal fat tissue, animal-free expansion in automated closed bioreactor systems and cryopreservation of the final product.

The active substance is aseptically prepared and *in vitro* expanded ASCs. The final product is provided as a cryopreserved suspension of 22 million ASCs/mL in Cryostor® 10. Total volume: 1.2 mL per vial. The excipient Cryostor® 10 holds 10% DMSO.

Baseline

Standard care burn depth evaluation is performed by clinical evaluation, and is guided by routine Laser Doppler Imaging (LDI)-scan. Baseline clinical photographs are taken. A blood sample is drawn and used as reference to subsequently detect possible donor-specific HLA antibodies in the recipient (by comparing to a repeated blood sample in relation to planned dressing change (at day 5, 10 and 14 post-intervention) and outpatient follow up). Right before the intervention with injections, a baseline biopsy (taken as two separate 4-mm punch biopsies; one for tissue homogenization and the other for microscopy), is taken centrally from the burn at the time of intervention. Following the unblinding of the study, the histological examination of the baseline biopsy is used to confirm the burn depth histologically.

Endpoints

The following endpoints are measured on deep partial-thickness burns; randomized into a homogenous pair of 6 x 6 cm treatment- / control areas.

*1. The degree of healing*

The *primary* endpoint is an evaluation of the treated burn's degree of healing, expressed as the *percentage reduction in wound surface area = (open wound area/initial wound area) x 100%,* on the 14th post-interventional day, evaluated by both contact- and non-contact planimetry.

Measurements of wound surface area have been shown to be a valid and accurate indicator of wound progress and allows assessment of treatment efficacy. Wound tracing is a reliable two-dimensional method of recording wound area that is easy to use and requires no special skills. When used in combination with planimetric area calculation it has good validity for shallow, pear-shaped wounds without undermined margins. The degree of burn wound healing in our study is defined as the percentage of the initial known wound area (baseline 6 x 6 cm) closed by re-epithelialization (characterized clinically by a dry, glistening, silvery surface, free of eschar); 100% re-epithelialization representing a fully healed wound. This is objectivised by two validated measurement techniques:

*Contact planimetry* - is performed by tracing the wound's perimeter onto a medical grade, sterile, transparent wound measurement film with a fine-tipped permanent marker pen. The wound surface area is then automatically and digitally calculated, by retracing the outline onto a digital tablet (Visitrak Digital, Smith and Nephew, Auckland, New Zealand). The produced visible record of the wound dimension can be used for documentation. See section *Wound measurement techniques* for further details on the Visitrak System

*Non-contact planimetry* (Photodigital planimetry software) - With standardised lighting and camera angle, a high-resolution, 2-dimensional, digital photograph of the wound is taken in the proximity of a ruler, to allow for distance-calibration. The image is then uploaded to computer software (ImageJ®). Following calibration and wound tracing from the digital photograph using either a mouse or digital pen on a digital tablet, the software automatically and accurately calculates the wound area.

*2. The rate of healing*

The *secondary* endpoint is an estimation of the burn's rate of healing (defined as the percentage reduction in wound surface area over time), evaluated by serial planimetric measurements (in relation to dressing changes) at day 5, 10 and 14(last measurement corresponds to the abovementioned), calculated as: *n* % healed per day.

*3. The degree of burn necrosis, inflammation, revascularisation, fibrosis and regeneration of normal structural-anatomy of the skin*

The *tertiary* endpoint is an assessment of the treated burn's degree of necrosis, inflammation, vascularity/neovascularisation, collagen organisation/fibrosis, regeneration of skin appendages and the restoration of normal structural skin anatomy. This is evaluated by a histological and immunohistochemical examination of a biopsy (taken as two separate 4-mm punch biopsies; one for tissue homogenization and the other for microscopy), taken from the center of the burn, on the 14th post-interventional day, compared to the baseline biopsy taken on day 0 (right before the intervention). From the aforementioned homogenized biopsies, the study subject's cellular response of cytokines, pro as well as anti-inflammatory and regenerative growth hormones and other relevant cellular responses of wound healing, are quantitated using immunoassays and qPCR arrays (as described in the section *Biopsy*).

*4. The blood perfusion*

The *quaternary* endpoint is an assessment of the dermal blood perfusion, corresponding closely to healing potential, which can be used to quantify the inflammatory response in a burn, This is evaluated by serial Laser Doppler Imaging (LDI) (moorLDLS-BI; Moor Instruments, Devon, UK), performed at baseline on day 0, after the intervention at 0, 30, 60, 90 and 120 min, and in relation to dressing changes on day 5, 10 and 14, calculated as: mean flux (mV). LDI-imaging is used as standard care in combination with clinical evaluation at our burn unit, to measure the healing potential of a burn, and as such, its burn depth (as described in section *Laser Doppler Imaging (LDI)*).

*5. The quality of healing (Main study only)*

The *quinary* endpoint is an assessment of the quality of healing of the treated burn, evaluated by measuring traditional skin quality parameters, such as: elasticity, hydration, pigmentation and transepidermal waterloss (TEWL - which is a measure for the quality of the barrier function of the skin). The objective measurements are performed on healed intact skin, by non-invasive probes connected to a multiparameter skin analysing device with analysing software (DermaLab Combo, Cortex Technology, Hadsund, Denmark) at an outpatient 3 and 6 months follow-up visit in the main study.

*Methods*

Study Plan

Patients included in the study (pilot- and the main study), receive a one-time administration/intervention consisting of local injections with ASCs and placebo, ≤ 36 hours post-burn (see **Fig. 11**). The intervention is performed on topically anaesthetised burns, using the local anaesthetic lidocaine-prilocaine 5% cream (Emla™). If needed, standard burn care morphica is supplemented orally or through the patient's standard peripheral venous line.

Hospital admission

Burn patients are initially hospitalized by the burn surgeon on call, according to standard practice. Primary standard care, which involves pain medication, cooling of burns with tap water, removing and deroofing of loose skin and blisters, respectively, cleansing and bacterial swab-test of burn wounds, is initially performed by the surgeon on call.

Intervention

The intervention is performed after baseline. Prior to and after to the intervention, a weight-adjusted dose of prophylactic intravenous Cefuroxime (or intravenous Clarithromycin) is administered through the patient's standard peripheral venous line.

Initially, a sterile medical grade skin staple is placed in each corner of the randomized squares of the anesthetized burn, to ensure delineation (is removed on the 14th post-interventional day) and photographs are taken for documentation.

A baseline biopsy (taken as two separate 4-mm punch biopsies; one for tissue homogenization and the other for microscopy), is hereafter taken from the centre of the square.

The intradermal injections are performed with the automatic, dermal injection gun Vital Injector 2 (EunSung Global Corporation, Seoul, South Korea) using a sterile 9 multi-needle system (31 Gauge). In order to account for the varying thickness of the skin, which varies between anatomical region, gender and age, a high frequency skin ultrasound probe, is used prior to the intervention, to give a high resolution, real-time scanning of the anaesthetized dermis. The measured thickness of the dermis will be used to set the target needle insertion/injection depth on the injection gun, to ensure intradermal drug delivery. In total, a dosage of 480 microliters of a 22 million ASCs/ml suspension or placebo is injected, perpendicular into the dermis, by placing the mouthpiece systematically onto the anaesthetized burn areas of 6 x 6 cm. The time of treatment is estimated to take approximately 3 minutes per treatment area, according to previous ex-vivo testing on discarded abdominal skin. A sterile plastic frame is used to guide injections.

After the intervention, the dermal blood perfusion in the treated burns is evaluated by LDI-imaging after 0, 30, 60, 90 and 120 min. In the same period, the areas are also observed for any local adverse reactions.

Finally, the burn wounds are applied sterile wound dressings and assessed for healing in relation to planned dressing change at day 5, 10 and 14. The need of conventional split-skin grafting will be assessed on the 14th day.

Day 14

- A bacterial swab-test from the treated burns is taken, using a sterile swab-stick to ensure that an unhealed wound is not caused by an infection (used as standard care in wound management).
- Following anaesthetization with Emla 5% cream, a biopsy (taken as two separate 4-mm punch biopsies; one for tissue homogenization and the other for microscopy), is taken from the centre of the area and will be compared to the baseline biopsy.
- Evaluation of the healing potential by an experienced burn surgeon.
- The skin staples are removed using a sterile, medical grade, staple remover.

3 and 6-months follow up (Main study only)

At an outpatient 3 and 6-months follow-up, the skin quality (the quinary endpoint) will be assessed. Clinical photos are taken and a blood sample is drawn (to test for possible alloantibodies). The recording of adverse events and reactions is done at each follow up.

Although there will be no HLA-tissue type matching between donors and the patients before treatment, HLA-tissue typing of all donors will be performed. Patient blood samples will be drawn at baseline, in relation to planned dressing change (at day 5, 10 and 14) and follow up and analyzed for the presence of allo-immunization by employing different immunoassays.

*Statistical analysis*

Statistical analysis is performed and graphs generated using GraphPad Prism 7 (GraphPad Software, La Jolla, USA). A paired Student's test will be used for all data comparisons. A p-value < 0.05 will be considered statistically significant.

*Acute burn injury assessment*

Clinical evaluation by burn experts is the standard care in burn depth assessment and remains the most frequent used technique, with an accuracy of 70-80% (Thatcher, J. E. et al., Adv. Wound Care 2016;5:360-378).

Clinical evaluation

Table 3 shows the clinical and histological features associated with the different degrees of burn depths.

Table 3

| Burn depth classification, associated clinical and histological features, and healing prognosis. | |
|---|---|
| **Superficial (Epidermal/ 1°)** | |
| Clinical appearance | Painful erythema; skin intact, no blistering. |
| Histology | Involve only the epidermis; basal membrane intact. |
| Prognosis | Heals in few days |
| **Superficial partial-thickness (Superficial-dermal/ 2°)** | |
| Clinical appearance | Blisters; underlying wound surface pink and wet that blanch on pressure; "sharp" sensation to needle prick test. Painful. |
| Histology | Involve the epidermis and the superficial part of the dermis; basal membrane partially destroyed. |
| Prognosis | Healing potential: < 14 days, usually with no scarring. |
| **Deep partial-thickness (Deep-dermal / 2°)** | |
| Clinical appearance | Blisters; underlying wound surface is usually mottled pink and white in color; fairly dry; less or no blanching on pressure; insensitive to needle prick test but sensitive to touch. Painful. |
| Histology | Involve the epidermis and the deep layer of the dermis; basal membrane completely destroyed. |
| Prognosis | Healing potential: $\geq$ 21 day, usually with scarring. |
| **Full-thickness (Subdermal / 3°)** | |
| Clinical appearance | Brown, black or white, leathery, firm, dry, anaesthetic (i.e not painful); in some cases, presenting with visible clotted vessels in the depth. |
| Histology | Involve all the layers of the skin and may include damage to subdermal structures such as: fat, muscle, cartilage and bone. |
| Prognosis | Healing potential: > 21 days, with considerable scarring. |

Biopsy

Histological examination of punch biopsies, is frequently considered as the "gold standard" of burn depth assessment.

To confirm the burn depth, burn lesions are biopsied right before the intervention on day 0 (baseline) and after 14 post-interventional days, using two 4.0 mm deposable skin punch biopsies; one for microscopy and the other for tissue homogenization (each 4 x 4 x 5 mm large tissue sample consisting of skin and subcutaneous tissue). The punch biopsy for microscopy is formalin fixed, paraffin embedded, cut, mounted on glass slides and stained with hematoxylin eosin (H&E) and examined in light microscope to determine burn depth (described as the observed anatomical depth of the necrotic tissue) and measure the quantitative degree of necrosis, inflammation, vascularity/neovascularisation, collagen organisation/fibrosis, and qualitatively regeneration of skin appendages and number of rete ridges to evaluate the restoration of normal structural skin anatomy.

Also, a panel of Immunohistochemical (IHC) staining is performed, such as CD31 staining for vascularity/neovascularisation; Myeloperoxidase for granulocytes (including neutrophils), monocytes and macrophages, Picrosirius red/Van Gieson staining for collagen organisation/fibrosis; Laminin antibody staining for basement membrane reformed between dermis and epidermis following re-epithelialization; (Cytokeratin Pan; rabbit anti-human cytokeratin for epidermal cells and adnexa); Caspase for apoptotic count and HMGB1 for necrosis. Histological images are captured using a digital scanning and image-analysis performed on IHC slides.

From the punch biopsy for homogenization, the study subject's cellular response of cytokines, pro as well as anti-inflammatory and regenerative growth hormones and other relevant cellular responses of wound healing, are quantitated using immunoassays and qPCR arrays.

Laser Doppler Imaging (LDI)

Laser Doppler Imaging measures dermal blood perfusion, which can be used to quantify the inflammatory response in a burn. The technique is based on the Doppler principle: a red visible laser light, scans the burn wound down to the superficial dermis where the scan head detects the laser light's reflection on the moving red blood cells, which exhibits a frequency change which is proportional to the amount of perfusion (flux, mV) in the tissue. Ceased dermal blood perfusion indicates necrotic tissue and consequently a deep burn with a low healing potential, because of the absent inflammatory response in the skin. A high dermal blood perfusion is typically observed for more superficial burns.

The LDI device generates a color-coded perfusion map that classifies burns into three healing potentials (i.e., burn depths): High, <14 days; Intermediate, 14-21 days; and low, >21 days. In addition, regions of interest may be targeted and their mean blood perfusion measured.

Wound measurement techniques

The Visitrak® system:

The validated system enables the measurement of the wound surface area, by tracing the wound's perimeter onto a transparent wound measurement film, with a fine-tipped permanent marker pen. Following retracing, onto the digital tablet (Visitrak Digital, Smith and Nephew, Auckland, New Zealand) it converts the line tracing into a digital area measurement, and can be used to calculate the percentage area change from the last area measurement as well as other regions of interest in the wound. The produced visible record of the wound dimension can be stored for documentation.

EXAMPLE 3

The perceived cell viability, recovery, proliferation (estimated confluency (e100%), lag-time/phase and PDt), Long-time cell viability (progression of cell death (Cytotoxicity)) and clogging assay described in Example 1 was repeated (n=3 donors) with the study drug in Example 2 (CSCC_ASC2210), using a 9-pin needle head. The results are shown in Figs. 12-15.

LIST OF REFERENCES

Each reference listed below or cited elsewhere herein is hereby specifically incorporated by reference, in its entirety.

Abo-Elkheir W, et al. Role of cord blood and bone marrow mesenchymal stem cells in recent deep burn: a case-control prospective study. Am J Stem Cells 2017;6(3):23-35.

Amer MH, et al. Translational considerations in injectable cell-based therapeutics for neurological applications: concepts, progress and challenges. Regenerative Medicine 2017;2:23.

Francis E, et al. The effects of stem cells on burn wounds: a review. Int J Burns Trauma 2019;9(1):1-12.

Fu X, et al. Enhanced wound-healing quality with bone marrow mesenchymal stem cells autografting after skin injury. Wound Rep Reg 2006;14:325-335.

Golchin A, et al. The Clinical Trials of Mesenchymal Stem Cell Therapy in Skin Diseases: An Update and Concise Review. Curr Stem Cell Res Ther 2019;14(1):22-33.

Isakson M, et al. Mesenchymal Stem Cells and Cutaneous Wound Healing: Current Evidence and Future Potential. Stem Cells Int. 2015; 2015: 831095.

Kondziolka D, et al. Injection parameters affect cell viability and implant volumes in automated cell delivery for the brain. Cell Transplant. 2011;20:1901-1906.

Leoni G., et al. Preclinical development of an automated injection device for intradermal delivery of a cell-based therapy. Drug Delivery and Translational Research 2017;7:695-708.

Mahla RS. Stem cells application in regenerative medicine and disease therapeutics. International Journal of Cell Biology 2016;7:19.

Mansilla E, et al. Cadaveric bone marrow mesenchymal stem cells: first experience treating a patient with large severe burns. Burns & Trauma 2015;3:17.

Rasulov MF, et al. First Experience in the Use of Bone Marrow Mesenchymal Stem Cells for the Treatment of a Patient with Deep Skin Burns. Cell Technologies in Biology and Medicine 2005; 1(1):141-144.

Sah SK, et al. Enhanced therapeutic effects of human mesenchymal stem cells transduced with superoxide dismutase 3 in a murine atopic dermatitis-like skin inflammation model. Allergy 2018;73(12):2364-2376.

EU Clinical Trials Register (EudraCT) number 2018-002870-27

WO 2006/074075 A2 (Primegen Biotech LLC)

WO 2014/203267 (Kasiak Research Pvt. Ltd.)

WO 2016/054592 A1 (Cytori Therapeutics Inc.)

WO 2017/068140 (Rigshospitalet)

WO 2017/144552 A1 (Centauri Biotech S.L.)

WO 2007/042818 A1 (Vuppalapati Gunasekar)

US 2007/0274967 AA

## Claims

1. A composition comprising a suspension of human stem cells for use in the treatment of a skin lesion in a human subject, comprising administering the composition to the skin lesion by intradermal or transdermal injection using one or more injection needles inserted into the skin at an angle of at least about 60 degrees to the skin surface.

2. The composition for the use according to any one of the preceding claims, wherein the human stem cells are mesenchymal stem cells (MSCs), optionally derived from adipose tissue, bone marrow, umbilical cord tissue and/or umbilical cord blood.

3. The composition for the use according to any one of the preceding claims, wherein the stem cells are adipose tissue-derived mesenchymal stem cells (ASCs).

4. The composition for the use according to any one of the preceding claims, wherein the skin lesion is selected from a wound, an ulcer, a scar and a rash.

5. The composition for the use according to any one of the preceding claims, wherein the skin lesion is a burn wound.

6. The composition for the use according to claim 5, wherein the burn wound has not been subjected to prior escharectomy or necrectomy.

7. The composition for the use according to any one of claims 5 and 6, wherein the burn wound is a partial-thickness burn wound, such as a deep partial-thickness burn wound at risk for progression into a full-thickness burn wound, or a superficial partial-thickness burn wound at risk for progressing into at least a deep-partial-thickness burn wound.

8. The composition for the use according to any one of the preceding claims, wherein the one or more injection needles are inserted into the skin at an angle of at least about 80 degrees to the skin surface, such as at least about 85 degrees to the skin surface, such as substantially perpendicular to the skin surface.

9. The composition for the use according to any one of the preceding claims, wherein the composition is administered at between 2 to 27 needle insertion sites per $cm^2$ of skin, such as between 3 and 18 insertion sites per $cm^2$ of skin, such as between 5 and 15 insertion sites per $cm^2$ of skin, such as between 7 and 11 insertion sites per $cm^2$ of skin.

10. The composition for the use according to any one of the preceding claims, wherein the composition is administered using a delivery device comprising plurality of substantially parallel injection needles, wherein the device is adapted to

   deliver a liquid formulation, optionally a viscous liquid formulation, through the plurality of substantially parallel injection needles when inserted into the skin, and
   keep the skin area in and around the insertion site of each injection needle taut during at least the needle insertion and injection phases, optionally by creating a vacuum, suction or mechanical force to gently lift the skin towards a surface comprising an opening from which the injection needle emerges.

11. The composition for the use according to any one of claims 10 to 11, comprising administering the composition by a method comprising the steps of:

   (a) applying the device to a predetermined area of the skin, wherein the device is loaded with a volume of the composition and activated to create a vacuum, suction or mechanical force to gently lift the skin towards a plurality of surfaces, each surface comprising an opening from which an injection needle can emerge;
   (b) activating the device to

      iii. insert the tip of each injection needle into a predetermined injection needle insertion/injection depth in the skin; and
      iv. inject from each injection needle a predetermined volume of the composition;

   (c) optionally, repeating steps (a) and (b) at an adjacent or other predetermined area of the skin.

12. The composition for the use according to any one of the preceding claims, wherein the composition is administered

   (a) at a needle insertion/injection depth of at least about 1 mm from the skin surface, such as at least 1.5 mm from the skin surface;
   (b) at a volume per injection needle insertion site of at least about 0.5 microliter ($\mu$L), at most about 20 $\mu$L, or both;
   (c) at an injection rate per needle of no more than about 0.5 mL/min, such as no more than about 0.4 mL/min, such as no more than about 0.3 mL/min, such as no more than about 0.25 mL/min, such as no more than about 0.22 mL/min;
   (d) with a needle latency in the skin for a period of at least 3 seconds, such as at least 4 seconds, such as at least 5 seconds, such as at least 6 seconds after injection; and/or
   (e) via one or more injection needles having an inner diameter of at least about 0.1 mm, at most about 0.5 mm, or both, and a length of at most about 3 cm, such as a length of at most 2 cm, such as a length of at most about 1.8 cm.

13. The composition for the use according to any one of the preceding claims, wherein the stem cells are ASCs and the composition is administered at a dosage of from about $1 \times 10^4$ to about $1 \times 10^6$ ASCs per $cm^2$.

**14.** The composition for the use according to any one of the preceding claims, wherein the stem cells are ASCs and the composition is prepared by suspending the ASCs in a protein-free cryoprotectant at a concentration of at least about $1 \times 10^7$ stem cells per mL, wherein the protein-free cryoprotectant comprises dimethylsulphoxide (DMSO) at a concentration of about 5% to about 15% (v/v), such as about 5% or about 10% (v/v).

**15.** The composition for the use according to any one of the preceding claims, wherein the composition is administered by intradermal injection.

FIG. 1

A) Viability

B) Recovery

C) Metabolism

FIG. 2

## A) Living cells at 12 h

## B) Cytotoxicity pin (12h)

FIG. 3

A) Adherence at 12h

B) lagtime

C) e100%

FIG. 4

A) **PDt**

B) **PDt**

FIG. 5

IDO

FIG. 6

A) **9 pin clot**

B) **5 pin clot**

FIG. 7

### A) 9 pin Pierce MB

### B) 9 pin tissue MB

### C) 9 pin reflux MB

FIG. 8

A) **5 pin piercings MB**

B) **5 pin tissue MB**

C) **5 pin reflux MB**

FIG. 9

FIG. 10

# FIG. 11

FIG. 12

A) **Viability**

B) **Recovery**

FIG. 13

A) e100%

B) Lag-phase

C) PDT max

FIG. 14

Progression of Cell death (cytotoxicity)

A) Donor 1

B) Donor 2

C) Donor 3

Cell death (number/well)

Time (24 hours)

D) Cytotoxicity pin (24h)

E) Cytotoxicity pin (24h)

Dead cells/well

baseline 9 pin

FIG. 15

**9 pin clot**

FIG. 16

A)

16 mm outer L & W

5 mm between needles

14 mm inner L & W

B)

7 mm between outermost needles

7 mm between outermost & central needle

13 mm outer L & W

11 mm inner L & W

## EUROPEAN SEARCH REPORT

**Application Number**

EP 20 17 5024

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/054592 A1 (CYTORI THERAPEUTICS INC [US]) 7 April 2016 (2016-04-07) | 1-15 | INV.<br>A61K35/28<br>A61K9/00<br>A61P17/02 |
| Y | * claims 1-85 * | 1-15 | |
| X,D | WO 2006/074075 A2 (PRIMEGEN BIOTECH LLC [US]; SAYRE CHAUNCEY B [US] ET AL.) 13 July 2006 (2006-07-13)<br>* paragraphs [0058] - [0061] *<br>* paragraphs [0085] - [0090] * | 1-15 | |
| X | WO 97/41208 A1 (UNIV CASE WESTERN RESERVE [US]; SORRELL J MICHAEL [US] ET AL.) 6 November 1997 (1997-11-06)<br>* example 5 * | 1-15 | |
| X | FU X ET AL: "Enhanced wound-healing quality with bonw marrow mesenchymal stem cells autografting after skin injury", WOUND REPAIR AND REGENERATION, MOSBY-YEAR BOOK, ST. LOUIS, MO, US, vol. 14, no. 3, 1 May 2006 (2006-05-01), pages 325-335, XP008124332, ISSN: 1067-1927, DOI: 10.1111/J.1743-6109.2006.00128.X [retrieved on 2006-06-28] *Animal experiments*; page 327 | 1-15 | TECHNICAL FIELDS SEARCHED (IPC)<br><br>A61K<br>A61P |

-/--

The present search report has been drawn up for all claims

| Place of search | Date of completion of the search | Examiner |
|---|---|---|
| Munich | 1 October 2020 | Schnack, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 17 5024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | LEONI GIULIA ET AL: "Preclinical development of an automated injection device for intradermal delivery of a cell-based therapy", DRUG DELIVERY AND TRANSLATIONAL RESEARCH, SPRINGER, GERMANY, vol. 7, no. 5, 15 August 2017 (2017-08-15), pages 695-708, XP036307259, ISSN: 2190-393X, DOI: 10.1007/S13346-017-0418-Z [retrieved on 2017-08-15] * the whole document * | 1-15 | |
| X | H Karimi ET AL: "BURN WOUND HEALING WITH INJECTION OF ADIPOSE-DERIVED STEM CELLS: A MOUSE MODEL STUDY", , 1 March 2014 (2014-03-01), pages 44-49, XP055735390, Retrieved from the Internet: URL:https://www.ncbi.nlm.nih.gov/pmc/artic les/PMC4158446/pdf/Ann-Burns-and-Fire-Disa sters-27-44.pdf [retrieved on 2020-09-30] | 1-15 | |
| Y | * page 45 * | 1-15 | TECHNICAL FIELDS SEARCHED (IPC) |
| X | PHILIPPE FOUBERT ET AL: "Adipose-Derived Regenerative Cell Therapy for Burn Wound Healing: A Comparison of Two Delivery Methods", ADVANCES IN WOUND CARE, vol. 5, no. 7, 1 July 2016 (2016-07-01), pages 288-298, XP055735397, ISSN: 2162-1918, DOI: 10.1089/wound.2015.0672 | 1-15 | |
| Y | * abstract * | 1-15 | |

-/--

The present search report has been drawn up for all claims

| Place of search | Date of completion of the search | Examiner |
|---|---|---|
| Munich | 1 October 2020 | Schnack, Anne |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

## EUROPEAN SEARCH REPORT

Application Number

EP 20 17 5024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHIN-JUNG FENG ET AL: "Adipose-derived stem cells-induced burn wound healing and regeneration of skin appendages in a novel skin island rat model :", JOURNAL OF THE CHINESE MEDICAL ASSOCIATION, vol. 82, no. 8, 1 August 2019 (2019-08-01) , pages 635-642, XP055735407, HK ISSN: 1726-4901, DOI: 10.1097/JCMA.0000000000000134 | 1-15 | |
| Y | * the whole document * ----- | 1-15 | |

TECHNICAL FIELDS
SEARCHED     (IPC)

The present search report has been drawn up for all claims

| Place of search | Date of completion of the search | Examiner |
|---|---|---|
| Munich | 1 October 2020 | Schnack, Anne |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 3

## ANNEX TO THE EUROPEAN SEARCH REPORT
## ON EUROPEAN PATENT APPLICATION NO.

EP 20 17 5024

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2020

| Patent document cited in search report | | | Publication date | Patent family member(s) | | | Publication date |
|---|---|---|---|---|---|---|---|
| WO 2016054592 | A1 | | 07-04-2016 | CA | 2963468 | A1 | 07-04-2016 |
| | | | | EP | 3200843 | A1 | 09-08-2017 |
| | | | | JP | 2017530151 | A | 12-10-2017 |
| | | | | US | 2017296697 | A1 | 19-10-2017 |
| | | | | WO | 2016054592 | A1 | 07-04-2016 |
| WO 2006074075 | A2 | | 13-07-2006 | AU | 2005322873 | A1 | 13-07-2006 |
| | | | | CA | 2592840 | A1 | 13-07-2006 |
| | | | | EP | 1830862 | A2 | 12-09-2007 |
| | | | | JP | 2008526762 | A | 24-07-2008 |
| | | | | US | 2006147430 | A1 | 06-07-2006 |
| | | | | WO | 2006074075 | A2 | 13-07-2006 |
| WO 9741208 | A1 | | 06-11-1997 | AU | 2808397 | A | 19-11-1997 |
| | | | | CA | 2253724 | A1 | 06-11-1997 |
| | | | | EP | 0953040 | A1 | 03-11-1999 |
| | | | | JP | 2000508922 | A | 18-07-2000 |
| | | | | US | 6497875 | B1 | 24-12-2002 |
| | | | | WO | 9741208 | A1 | 06-11-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017068140 A
- WO 2014203267 A
- WO 2017144552 A
- WO 2006074075 A
- WO 2016054592 A
- US 20070274967 A
- WO 2007042818 A
- US 20070248574
- WO 2016054592 A1
- WO 2011047289 A1
- US 20120251504 A
- US 20130183273
- US 20110151556 A
- WO 04072273 A1
- US 8021876 B

- WO 201003369 A1
- WO 2012014247 A1
- WO 2009052132 A
- US 20130230924
- US 20050054093 A
- US 20130065302
- WO 2017144552 A1
- WO 2017068140 A1
- WO 2000002572 A1
- WO 2010064054 A1
- WO 2006074075 A2
- WO 2007042818 A1
- US 20070274967A A

**Non-patent literature cited in the description**

- **PECK.** *Burns,* 2011, vol. 37, 1087-100
- **BOURIN et al.** *Cytotheraphy,* June 2013, vol. 15 (6), 641-648
- **TOMA et al.** *Nat Cell Biol.,* 2001, vol. 3 (9), 778-84
- **NOWAK et al.** *Methods Mol Biol.,* 2009, vol. 482, 215-32
- **VAN DER VEEN et al.** *Cell Transplant,* 2012, vol. 21 (5), 933-42
- **GNECCHI ; MELO.** *Methods Mol Biol,* 2009, vol. 482, 281-94
- **NODA et al.** *Sci Rep,* 2019, vol. 9, 5430
- **COROTCHI et al.** *Stem Cell Research and Therapy,* 2013, vol. 4, 81
- **REDDY et al.** *Methods Mol Biol.,* 2007, vol. 407, 149-63
- **HUSSAIN et al.** *Cell Biol Int.,* 2012, vol. 36 (7), 595-600
- **PHAM et al.** *Journal of Translational Medicine,* 2014, vol. 12, 56
- **LEE et al.** *Blood,* 2004, vol. 103 (5), 1669-1675
- **DAYEM et al.** *Int J Mol Sci.,* April 2019, vol. 20 (8), 1922
- **HAACK-SORENSEN et al.** *Scand J Clin Lab Invest.,* July 2018, vol. 78 (4), 293-300
- **HAACK-SØRENSEN et al.** *J Transl Med.,* 16 November 2016, vol. 14 (1), 319
- **PANES et al.** *Lancet,* 2016, vol. 388 (10051), 1281-90

- **LIN et al.** *Cell Transplant.,* March 2017, vol. 26 (3), 449-460
- **CHEN et al.** *Br J Pharmacol,* 2009, vol. 158 (5), 1196-209
- **SHARMA et al.** *Advanced Wound Care,* 2019, vol. 8 (12), 655
- **LU et al.** *Stem Cells International,* vol. 2018
- **PAUTI et al.** *EBioMedicine,* January 2018, vol. 27, 225-236
- **REN et al.** *Stem Cells,* August 2009, vol. 27 (8), 1954-62
- **MEHANNA et al.** *Biomed Res Int.,* 2015, vol. 2015, 846062
- **FALANGA et al.** *Tissue Eng.,* 2007, vol. 13, 1299-1312
- **SAH et al.** *Allergy,* December 2018, vol. 73 (12), 2364-2376
- **GERLACH et al.** *Burns,* 2011, vol. 37, e19-e23
- **MORONKEJI et al.** *J. Control. Release,* 2018, vol. 265, 102-112
- **GENINA et al.** *Proc. SPIE,* 2004, 5486
- **GUPTA et al.** *Biomaterials,* 2011, vol. 32, 6823-6831
- **GRAMLICH et al.** *Sci. Rep.,* 2016, vol. 6
- **BAUST et al.** *In Vitro Cellular and Developmental Biology - Animal,* 2017, vol. 53, 855-871
- **DAVIES et al.** *Cryobiology,* 2014, vol. 69, 342-347

- **THATCHER, J. E. et al.** *Adv. Wound Care,* 2016, vol. 5, 360-378
- **ABO-ELKHEIR W et al.** Role of cord blood and bone marrow mesenchymal stem cells in recent deep burn: a case-control prospective study. *Am J Stem Cells,* 2017, vol. 6 (3), 23-35
- **AMER MH et al.** Translational considerations in injectable cell-based therapeutics for neurological applications: concepts, progress and challenges. *Regenerative Medicine,* 2017, vol. 2, 23
- **FRANCIS E et al.** The effects of stem cells on burn wounds: a review. *Int J Burns Trauma,* 2019, vol. 9 (1), 1-12
- **FU X et al.** Enhanced wound-healing quality with bone marrow mesenchymal stem cells autografting after skin injury. *Wound Rep Reg,* 2006, vol. 14, 325-335
- **GOLCHIN A et al.** The Clinical Trials of Mesenchymal Stem Cell Therapy in Skin Diseases: An Update and Concise Review. *Curr Stem Cell Res Ther,* 2019, vol. 14 (1), 22-33
- **ISAKSON M et al.** Mesenchymal Stem Cells and Cutaneous Wound Healing: Current Evidence and Future Potential. *Stem Cells Int,* 2015, vol. 2015, 831095
- **KONDZIOLKA D et al.** Injection parameters affect cell viability and implant volumes in automated cell delivery for the brain. *Cell Transplant,* 2011, vol. 20, 1901-1906
- **LEONI G. et al.** Preclinical development of an automated injection device for intradermal delivery of a cell-based therapy. *Drug Delivery and Translational Research,* 2017, vol. 7, 695-708
- **MAHLA RS.** Stem cells application in regenerative medicine and disease therapeutics. *International Journal of Cell Biology,* 2016, vol. 7, 19
- **MANSILLA E et al.** Cadaveric bone marrow mesenchymal stem cells: first experience treating a patient with large severe burns. *Burns & Trauma,* 2015, vol. 3, 17
- **RASULOV MF et al.** First Experience in the Use of Bone Marrow Mesenchymal Stem Cells for the Treatment of a Patient with Deep Skin Burns. *Cell Technologies in Biology and Medicine,* 2005, vol. 1 (1), 141-144
- **SAH SK et al.** Enhanced therapeutic effects of human mesenchymal stem cells transduced with superoxide dismutase 3 in a murine atopic dermatitis-like skin inflammation model. *Allergy,* 2018, vol. 73 (12), 2364-2376